# EUROPEAN PATENT SPECIFICATION

(11) **EP 1 165 113 B1**
(45) Date of publication and mention of the grant of the patent: **11.06.2008**
(21) Application number: 00921827.2
(22) Date of filing: 05.04.2000
(51) Int. Cl.: A61P 5/50, A61K 38/18

(54) **USE OF ErbB RECEPTOR LIGANDS IN TREATING DIABETES**
VERWENDUNG VON ERBB-REZEPTORLIGANDEN ZUR BEHANDLUNG VON DIABETES
UTILISATION DE LIGANDS RECEPTEURS DE ErbB DANS LE TRAITEMENT DU DIABETE

(30) Priority: 06.04.1999 US 128017 P
(43) Date of publication of application: 02.01.2002
(73) Proprietor: GENENTECH, INC., South San Francisco, CA 94080-4990 (US)
(72) Inventor: HUANG, Xiaojian, Palo Alto, CA 94303 (US); STEWART, Timothy, Andrew, San Francisco, CA 94114 (US)
(74) Representative: Kiddle, Simon John
(86) International application number: PCT/US2000/009240
(87) International publication number: WO 2000/059525

(56) References cited:
- WO-A-00/77195
- WO-A-95/19785
- HUDZIAK R M ET AL: "MONOCLONAL ANTIBODY HAS ANTIPROLIFERATIVE EFFECTS IN VITRO AND SENSITIZES HUMAN BREAST TUMOR CELLS TO TUMOR NECROSIS FACTOR" MOLECULAR AND CELLULAR BIOLOGY, AMERICAN SOCIETY FOR MICROBIOLOGY, WASHINGTON, US, vol. 9, no. 3, March 1989 (1989-03), pages 1165-1172, XP000918174 ISSN: 0270-7306
- WU X ET AL: "APOPTOSIS INDUCED BY AN ANTI-EPIDERMAL GROWTH FACTOR RECEPTOR MONOCLONAN ANTIBODY IN A HUMAN COLORECTAL CARCINOMA CELL LINE AND ITS DELAY BY INSULIN" JOURNAL OF CLINICAL INVESTIGATION, NEW YORK, NY, US, vol. 95, no. 4, 1 April 1995 (1995-04-01), pages 1897-1905, XP000645039 ISSN: 0021-9738
- BASELGA JOSE ET AL: "Receptor blockade with monoclonal antibodies as anti-cancer therapy." PHARMACOLOGY & THERAPEUTICS, vol. 64, no. 1, 1994, pages 127-154, XP009011842 ISSN: 0163-7258

## Description

### Field of the Invention

This invention relates to the use of heregulin in medicaments for treating diabetes and other conditions associated with pancreatic dysfunction.

### Background of the Invention

### The ErbB Receptor and Ligand Family

Transduction of signals that regulate cell growth and differentiation is regulated in part by phosphorylation of various cellular proteins. Protein tyrosine kinases are enzymes that catalyze this process. Receptor protein tyrosine kinases are believed to direct cellular growth via ligand-stimulated tyrosine phosphorylation of intracellular substrates. The ErbB receptor family belongs to the subclass I receptor tyrosine kinase superfamily and includes four distinct receptors including epidermal growth factor receptor (EGFR or ErbB1), ErbB2 (HER2 or p 185^{neu}). ErbB3 (HER3), and ErbB4 (HER4 or tyro2).

EGFR or ErbB1 has been causally implicated in human malignancy and, in particular, increased expression of this gene has been observed in more aggressive carcinomas of the breast, bladder, lung and stomach. Increased EGFR expression has been reported to be often associated with increased production of the EGFR ligand, transforming growth factor-alpha (TGF-alpha), by the same tumor cells, resulting in receptor activation by an autocrine stimulatory pathway. [Baselga et al.. Pharmac. Ther-. 64:127-154 (1994)]. Monoclonal antibodies directed against the EGFR, or its ligands TGF-alpha and EGF. have been evaluated as therapeutic agents in the treatment of such malignancies. [See, e.g., Baselga et al., *supra:* Masui et al., Cancer Research 44: 1002-1007 (1984); Wu et al., J. Clin. Invest. 95:1897-1905 (1995)].

The second member of the class I subfamily, p185^{neu}, was originally identified as the product of the transforming gene from neuroblastomas of chemically treated rats. The *neu* gene (also called *erb*B2 and HER2) encodes a 185 kDa receptor protein tyrosine kinase. Amplification and/or overexpression of the human ErbB2 gene correlates with a poor prognosis in breast and ovarian cancers. [Slamon et al.. Science 235:177-182 (1987); and Slamon et al., Science 244:707-712 (1989); US Patent 4,968.603]. Overexpression of ErbB2 has been observed with other carcinomas including carcinomas of the stomach, endometrium, salivary gland, lung, kidney, colon and bladder. Accordingly, Slamon et al. in U.S. Patent No. 4.968.603 describe and claim various diagnostic assays for determining ErbB2 gene amplification or expression in tumor cells.

Antibodies directed against the rat p 185 ^{neu} and human ErbB2 gene products have been described. For instance. Drebin et al., Cell 41:695-706 (1985); Meyers et al., Methods Enzym. 198:277-290 (1991); and WO 94/22478 describe antibodies directed against the rat gene product, p185^{neu}. Hudziak et al., Mol. Cell. Biol. 9:1165-1172 (1989) describe the generation of a panel of anti-ErbB2 antibodies which were characterized using the human breast tumor cell line SKBR3. Other anti-ErbB2 antibodies have also been reported in the literature. [See. e.g., US Patents 5.821.337 and 5.783.186: WO 94/00136; Tagliabue et al.. Int. J. Cancer 47:933-937 (1991): McKenzie et al.. Oncogene 4:543-548 (1989): Maier et al.. Cancer Res. 51:5361-5369 (1991): Bacus et al.. Molecular Carcinogenesis 3:350-362 (1990): Xu et al., Int. J. Cancer 53:401-408 (1993); Kasprzyk et al.. Cancer Research 52:2771 -2776 (1992): Hancock et al.. Cancer Research 51:4575-4580 (1991); Shawver et al.. Cancer Research 54:1367-1373 (1994): Arteaga et al.. Cancer Research 54:3758-3765 (1994); Harwerth et al.. J. Biol. Chem. 267:15160-15167 (1992)].

A further related gene, called *erbB3* or HER3. has also been described. See U.S. Patent No. 5.183,884 and 5.480.968: Kraus et al.. Proc. Natl. Acad. Sci. USA 86:9193-9197 (1989): EP patent application number 444.961A1; and Kraus et al.. Proc. Natl. Acad. Sci. USA 90:2900-2904 (1993). Kraus et al. (1989) discovered that markedly elevated levels of *erb*B3 mRNA were present in certain human mammary tumor cell lines indicating that *erb*B3*,* like *erb*B1 and *erb*B2*,* may play a role in human malignancies. Also, Kraus et al.. *supra* (1993) showed that EGF-dependent activation of the ErbB3 catalytic domain of a chimeric EGFR/ErbB3 receptor resulted in a proliferative response in transfected NIH-3T3 cells. This is now believed to be the result of endogenous ErbB1 or ErbB2 in NIH-3T3. Furthermore, these researchers demonstrated that some human mammary tumor cell lines display a significant elevation of steady-state ErbB3 tyrosine phosphorylation further indicating that this receptor may play a role in human malignancies. The role of *erb*B3 in cancer has been explored by others. It has been found to be overexpressed in breast [Lemoine et al.. Br. J. Cancer 66:1116-1121 (1992)], gastrointestinal [Poller et al.. J. Pathol. 168:275-280 (1992), Rajkumer et al., J. Pathol. 170:271-278 (1993), and Sanidas et al., Int. J. Cancer 54:935-940 (1993)], and pancreatic cancers [Lemoine et al., J. Pathol. 168:269-273 (1992); Friess et al., Clinical Cancer Research 1:1413-1420 (1995)].

The class I subfamily of epidermal growth factor receptor protein tyrosine kinases has been further extended to include the ErbB4 receptor. [See EP patent application number 599.274: Plowman et al.. Proc. Natl. Acad. Sci. USA 90:1746-1750 (1993): and Plowman et al.. Nature 366:473-475 (1993)]. Plowman et al. found that increased ErbB4 expression closely correlated with certain carcinomas of epithelial origin, including breast adenocarcinomas. Diagnostic methods for detection of human neoplastic conditions (especially breast cancers) which evaluate ErbB4 expression are described in EP Appln. No. 599,274.

Various ligands which bind and/or activate such ErbB receptors have been described in the literature. The ligands include the polypeptides referred to as EGF [Savage et al., J. Biol. Chem. 247:7612-7621 (1972)], TGF-alpha [Marquardt et al., Science 223:1079-1082 (1984)], amphiregulin [Shoyab et al., Science 243:1074-1076 (1989); Kimura et al., Nature 348:257-260 (1990); Cook et al., Mol. Cell. Biol. 11:2547-2557 (1991)], heparin-binding EGF (HB-EGF) [Higashiyama et al., Science 251:936-939 (1991)], betacellulin [Shing et al., Science 259:1604-1607 (1993)], and epiregulin [Toyoda et al., J. Biol. Chem. 270:7495-7500 (1995)]. ErbB1 is bound by six different ligands; epidermal growth factor (EGF), TGF-alpha, amphiregulin, HB-EGF. betacellulin, and epiregulin. [See also, e.g., Groenen et al., Growth Factors 11:235-257 (1994)].

A family of heregulin proteins resulting from alternative splicing of a single gene are ligands for ErbB3 and ErbB4. As discussed further below, the heregulin family includes NDFs. GGFs, and ARIA. [Groenen et al., Growth Factors 11:235-257 (1994): Lemke. Molec. & Cell. Neurosc. 7:247-262 (1996): Lee et al.. Pharm. Rev. 47:51-85 (1995)]. Further ErbB ligands have been identified- neuregulin-2 (NRG-2) which is reported to bind either ErbB3 or ErbB4 [Chang et al., Nature 387:509-512 (1997); Carraway et al., Nature 387:512-516 (1997)] and neuregulin-3 which binds ErbB4 [Zhang et al., Proc. Natl. Acad. Sci. 94:9562-9567 (1997)]. HB-EGF, betacellulin, and epiregulin also bind to ErbB4.

While EGF and TGF-alpha do not bind ErbB2, EGF stimulates ErbB1 and ErbB2 to form a heterodimer, which activates ErbB1 and results in transphosphorylation of ErbB2 in the heterodimer. Dimerization and/or transphosphorylation appears to activate the ErbB2 tyrosine kinase. Likewise, when ErbB3 is co-expressed with ErbB2, an active signaling complex is formed and antibodies directed against ErbB2 are capable of disrupting the complex. [Sliwkowski et al., J. Biol. Chem. 269:14661-14665 (1994)]. Additionally, the affinity of ErbB3 for heregulin is increased to a higher affinity state when co-expressed with ErbB2. [Levi et al., J. Neuroscience 15:1329-1340 (1995); Morrisey et al., Proc. Natl. Acad. Sci. 92:1431-1435 (1995) and Lewis et al., Cancer Research 56:1457-1465 (1996) with respect to the ErbB2-ErbB3 protein complex]. ErbB4. like ErbB3. forms an active signaling complex with ErbB2. [Carraway et al., Cell 78:5-8 (1994)].

Holmes et al. isolated and cloned a family of polypeptide activators for the ErbB2 receptor which they called heregulin-alpha (HRG-alpha), heregulin-betal (HRG-betal), heregulin-beta2 (HRG-beta2), heregulin-beta2-like (HRG-beta2-like), and heregulin-beta3 (HRG-beta3). [See Holmes et al., Science 256:1205-1210 (1992); WO 92/20798: and U.S. Patent No. 5,367,060]. The 45 kDa polypeptide, HRG-alpha, was purified from the conditioned medium of the MDA-MB-231 human breast cancer cell line. These researchers demonstrated the ability of the purified heregulin polypeptides to activate tyrosine phosphorylation of the ErbB2 receptor in MCF7 breast tumor cells. Furthermore, the mitogenic activity of the heregulin polypeptides on SK-BR-3 cells (which express high levels of the ErbB2 receptor) was illustrated.

While heregulins are substantially identical in the first 213 amino acid residues, they are classified into two major types, alpha and beta, based on two variant EGF-like domains which differ in their C-terminal portions. Nevertheless, these EGF-like domains are identical in the spacing of six cysteine residues contained therein. Based on an amino acid sequence comparison, Holmes et al. found that between the first and sixth cysteines in the EGF-like domain. HRGs were 45% similar to heparin-binding EGF-like growth factor (HB-EGF), 35% identical to amphiregulin (AR), 32% identical to TGF-alpha, and 27% identical to EGF.

The 44 kDa neu differentiation factor (NDF), which is the rat equivalent of human HRG. was first described by Peles et al.. Cell. 69:205-216 (1992): and Wen et al., Cell, 69:559-572 (1992). Like the HRG polypeptides, NDF has an immunoglobulin (Ig) homology domain followed by an EGF-like domain and lacks a N-terminal signal peptide. Subsequently, Wen et al., Mol. Cell. Biol., 14(3):1909-1919 (1994) carried out "exhaustive cloning" to extend the family ofNDFs. This work revealed six distinct fibroblastic pro-NDFs. Adopting the nomenclature of Holmes et al., the NDFs are classified as either alpha or beta polypeptides based on the sequences of the EGF-like domains. These researchers conclude that different NDF isoforms are generated by alternative splicing and perform distinct tissue-specific functions. See also EP 505 148; WO 93/22424; and WO 94/28133 concerning NDF.

Falls et al., Cell, 72:801-815 (1993) describe another member of the heregulin family which they call acetylcholine receptor inducing activity (ARIA) polypeptide. The chicken-derived ARIA polypeptide stimulates synthesis of muscle acetylcholine receptors. See also WO 94/08007. ARIA is a type I heregulin with a beta type EGF domain.

Marchionni et al., Nature, 362:312-318 (1993) identified several bovine-derived proteins which they call glial growth factors (GGFs). These GGFs share the Ig-like domain and EGF-like domain with the other heregulin proteins described above, but also have an amino-terminal kringle domain. GGFs generally do not have the complete glycosylated spacer region between the Ig-like domain and EGF-like domain. Only one of the GGFs, GGFII, possessed a N-terminal signal peptide. See also WO 92/18627; WO 94/00140: WO 94/04560: WO 94/26298; and WO 95/32724 which refer to GGFs and uses thereof.

Ho et al., in J. Biol. Chem. 270(4):14523-14532 (1995), describe another member of the heregulin family called sensory and motor neuron-derived factor (SMDF). This protein has an EGF-like domain characteristic of all other heregulin polypeptides but a distinct N-terminal domain. The major structural difference between SMDF and the other heregulin polypeptides is that SMDF lacks the Ig-like domain and the "glyco" spacer characteristic of all the other heregulin polypeptides. Another feature of SMDF is the presence of two stretches of hydrophobic amino acids near the N-terminus.

While heregulin polypeptides were first identified based on their ability to activate the ErbB2 receptor (see Holmes et al., *supra),* it was discovered that certain ovarian cells expressing neu and neu-transfected fibroblasts did not bind or cross-link to NDF. nor did they respond to NDF to undergo tyrosine phosphorylation (Peles et al.. EMBO J. 12:961-971 (1993)). This indicated another cellular component was necessary for conferring full heregulin responsiveness. Carraway et al. subsequently demonstrated that ¹²⁵I-rHRG β 1₁₇₇₋₂₄₄ bound to NIH-3T3 fibroblasts stably transfected with bovine *erb*B3 but not to non-transfected parental cells. Accordingly, the investigators suggested that ErbB3 is a receptor for HRG and mediates phosphorylation of intrinsic tyrosine residues as well as phosphorylation of ErbB2 receptor in cells which express both receptors. Carraway et al.. J. Biol. Chem. 269(19):14303-14306 (1994). Sliwkowski et al., J. Biol. Chem. 269(20):14661-14665 (1994) found that cells transfected with ErbB3 alone show low affinities for heregulin, whereas cells transfected with both ErbB2 and ErbB3 show higher affinities.

This observation correlates with the "receptor cross-talking" described previously by Kokai et al., Cell 58:287-292 (1989): Stem et al., EMBO J. 7:995-1001 (1988); and King et al., 4:13-18 (1989). These researchers found that binding of EGF to the ErbB1 resulted in activation of the ErbB1 kinase domain and cross-phosphorylation of p185^{HER2}. This is believed to be a result of ligand-induced receptor heterodimerization and the concomitant cross-phosphorylation of the receptors within the heterodimer. [Wada et al., Cell 61:1339-1347 (1990)].

Plowman and his colleagues have similarly studied p 185^{HER4}/p185^{HER2} activation. They expressed p185^{HER2} alone, p185^{HER4} alone, or the two receptors together in human T lymphocytes and demonstrated that heregulin is capable of stimulating tyrosine phosphorylation of p 185^{HER4}, but could only stimulate p 185^{HER2} phosphorylation in cells expressing both receptors. [Plowman et al., Nature 336:473-475 (1993)].

### Other Biological Roles of ErbB Receptor Ligands

Other biological role(s) of various ErbB ligands have been investigated by several groups. For example, betacellulin has been reported to exhibit growth-promoting activity in vascular smooth muscle cells and retinal pigment epithelial cells. [Shing et al., *supra].* Falls et al., *supra,* found that ARIA plays a role in myotube differentiation, namely affecting the synthesis and concentration of neurotransmitter receptors in the postsynaptic muscle cells of motor neurons. Corfas and Fischbach demonstrated that ARIA also increases the number of sodium channels in muscle. [Corfas and Fischbach, J. Neuroscience, 13(5):2118-2125 (1993)]. It has also been shown that GGFII is mitogenic for subconfluent quiescent human myoblasts and that differentiation of clonal human myoblasts in the continuous presence of GGFII results in greater numbers of myotubes after six days of differentiation. [Sklar et al., J. Cell Biochem., Abst. W462, 18D. 540 (1994)]. See also WO 94/26298 published November 24. 1994.

Holmes et al., supra. found that HRG exerted a mitogenic effect on mammary cell lines (such as SK-BR-3 and MCF-7). The mitogenic activity of GGFs on Schwann cells has also been reported. [See. e.g., Brockes et al.. J. Biol. Chem. 255(18):8374-8377 (1980): Lemke and Brockes, J. Neurosci. 4:75-83 (1984); Brockes et al., J. Neuroscience 4(1):75-83 (1984): Brockes et al., Ann. Neurol. 20(3):317-322 (1986), Brockes. J., Methods in Enzym. 147:217-225 (1987) and Marchionni et al., *supra*]*.*

Pinkas-Kramarski et al. found that NDF seems to be expressed in neurons and glial cells in embryonic and adult rat brain and primary cultures of rat brain cells, and suggested that it may act as a survival and maturation factor for astrocytes. [Pinkas-Kramarski et al., PNAS, USA 91:9387-9391 (1994)]. Meyer and Birchmeier. PNAS, USA 91:1064-1068 (1994) analyzed expression of heregulin during mouse embryogenesis and in the perinatal animal using *in situ* hybridization and Rnase protection experiments. See also Meyer et al., Development 124(18):3575-3586 (1997). Similarly, Danilenko et al., Abstract 3101, FASEB 8(4-5):A535 (1994) and Danilenko et al.. Journal of Clinical Investigation 95(2):842-851 (1995), found that the interaction of NDF and the ErbB2 receptor is important in directing epidermal migration and differentiation during wound repair.

Ram et al., Journal of Cellular Physiology 163:589-596 (1995) evaluated the mitogenic activity of NDF on the immortalized human mammary epithelial cell line MCF-10A. Danilenko et al.. J. Clin. Invest. 95:842-851 (1995) investigated whether NDF would influence epidermal migration in an *in vivo* model of excisional deep partial-thickness wound repair. It is reported that there were no statistically significant differences in proliferating basal and superbasal keratinocytes in control wounds vs. wounds treated with rhNDF-α₂. Marikovsky et al., Oncogene 10:1403-141 (1995), studied the proliferative responses ofan aneuploid BALB/MK continuous keratinocyte cell line and evaluated the effects of α- and β- isoforms of NDF on epidermal keratinocytes.

The potential role(s) that the various ErbB ligands may play in pancreatic cell proliferation and differentiation has also been reported by several investigators. Islet cells (also referred to as Islets of Langerhans) in the pancreas are known to produce the hormones, insulin, and glucagon. Such islet cells are believed to be derived from stem cells in the fetal ductular pancreatic endothelium. [Pictet and Rutter, "Development of the embryonic pancreas", Endocrinology, Handbook of Physiology, 1972. American Physiological Society, Washington D.C.. pages 25-66]. In particular, during development, the pancreas forms a system of tubules composed of a single layer of undifferentiated cells, which may then differentiate into duct cells, acinar cells or islet cells. [See. e.g.. LeDouarin. Cell. 53:169-171 (1998): Teitelman. Recent Prog. Hormone Res., 47:259-297 (1991)].

There are several different types of islet cells which can be identified histologically, including cells referred to as alpha cells and beta cells. Insulin is synthesized in the pancreatic islet by the beta cells. In various circumstances, the islet beta cells may fail to secrete sufficient amounts of insulin, eventually leading to abnormally high levels of glucose in the blood (a condition often referred to as hyperglycemia). Control of insulin production at the cellular level is achieved in the beta cells through regulatory mechanisms operating at the transcriptional, translational, and post-translational levels. [Sjoholm. J. Int. Med., 239:211-220 (1996)]. Insulin has a variety of biological activities in mammals, some of which are tissue specific. For instance, insulin may enhance milk production in the mammary gland, stimulate fat synthesis in the liver, promote the transport of glucose into muscle tissue and stimulate growth of connective tissues.

Insulin deficiency in mammals can result in serious pathological conditions. For example, in Type I diabetes, the pancreas typically produces little or no insulin. Type I diabetes is generally characterized as a T cell mediated autoimmune disease in which pancreatic beta cells are typically destroyed. The disease usually affects children and adolescents, but may occur at any age. Various environmental triggers. e.g.. certain viruses or dietary components, have been proposed to initiate the autoimmune process, in which T cells are thought to play an important role. [Akerblom et al., Diabetes/Metabolism Reviews. 14:31-67 (1998)]. Susceptibility or resistance to Type 1 diabetes may also be dependent upon the genetic makeup of the individual. [Tisch et al., Cell. 85:291-297 (1996)]. For a general review, see Rabinovitch et al.. Prevention and Treatment of Diabetes and Its Complications, 82:739-755 (1998).

In the condition known as Type II diabetes, the pancreas will generally produce some insulin, but the amount secreted is insufficient for the mammal to maintain physiologically acceptable glucose levels. Type II diabetes is the more common type of diabetes and affects millions of individuals, many of whom are unaware that they have the condition. This type of diabetes is usually characterized by three separate but interrelated defects. An affected individual may have one or all of these defects and varying degrees. These defects are insulin resistance, impaired insulin secretion, and inappropriate release of glucose by the liver.

Yet another form of diabetes is referred to as gestational diabetes. This type of diabetes is typically a diabetic condition that is first diagnosed in an individual during pregnancy and resolves after delivery.

The further complications of such diabetic conditions are varied and include small and large-caliber blood vessel damage and peripheral nerve damage, which in turn can increase risks of heart attack, stroke, blindness and kidney failure.

Various investigators have reported on the effects of particular EGF, heregulin and heregulin-related polypeptides on islet cells. In WO 95/19785 published July 27, 1995, methods for treating diabetes mellitus are described wherein a combination of a gastrin/CCK receptor ligand and an EGF receptor ligand (e.g., TGF-alpha) are administered in amounts sufficient to effect differentiation of pancreatic islet precursor cells to mature insulin-secreting cells. WO 95/19785 teaches that the TGF-alpha polypeptide was not capable of stimulating differentiation of the islet precursor cells when administered alone.

In WO 97/17086 published May 15, 1997, it is reported that particular betacellulin proteins were capable of promoting differentiation of a pancreatic cell line, AR42J (rat cells derived from a chemically induced pancreatic tumor) into insulin-producing beta cells. In the WO 97/17086 application, it is also reported that other heregulin family members, like EGF, TGF-alpha and FGF, failed to effectively induce such differentiation in the AR42J cells. [See also, Ishiyama et al., Diabetologia, 41:623-628 (1998); Mashima et al., J. Clin. Invest., 97:1647-1654 (1996)]. The betacellulin proteins tested in such experiments, while showing some differentiation activity in the AR42J cells, did not have growth factor (proliferative) activity. [Ishiyama et al., *supra*.]

The effects ofsuch ligands on other pancreatic insulinoma cell lines have also been described in the literature. Huotari et al. report that betacellulin exhibited a mitogenic effect on INS-1 cells *in vitro,* while EGF, TGF-alpha and TGF-beta were inactive. [Huotari et al., Endocrinology, 139:1494-1499 (1998).] It was further reported that neither betacellulin, EGF, TGF-alpha or TGF-beta affected the insulin content of the INS-1 cells. The betacellulin had no mitogenic effect on RINm5F cells, whereas EGF and TGF-alpha were slightly mitogenic. [Huotari et al., *supra*.]

Watada et al. have investigated some of the transcription factors believed to be important for insulin gene expression in islet cells. [Watada et al., Diabetes 45:1826-1831 (1996).] In particular, Watada et al. examined the transcription factor PDX-1, a factor found to appear before insulin during ontogeny of the mouse pancreas and whose expression becomes restricted to pancreatic beta cells in the adult animal. The PDX-1 gene was introduced into α TC1.6 cells and the changes in the gene expression pattern were observed when the cells were treated with various growth factors. Watada et al. report that betacellulin was capable of inducing endogenous insulin and glucokinase gene expressions in the PDX-1-expressing α TC1.6 cells, but that the growth factors TFG-alpha, TFG- β, EGF, IGF-I and bFGF had no such effect.

WO96/30403 suggest that neuregulin may play a role in retinal cell survival and function, and propose the use of neuregulin in the treatment of diabetic retinopathy.

### Summary of the Invention

The present invention concerns compositions and uses for treating pancreatic dysfunction, particularly diabetes, in mammals. The invention is based, in part, on the identification of ErbB receptor ligands testing positive in various assays for the expression or secretion of insulin or expression of transcription factors or markers unique for pancreatic beta cells. Thus, the ErbB receptor ligands described herein are thought to be useful drugs for the treatment of conditions associated with insulin deficiency.

In one embodiment, the invention provides a use for treating conditions in mammals associated with pancreatic dysfunction, and particularly treating conditions in mammals associated with impaired beta cell function. In a preferred embodiment, the condition being treated is diabetes, and even more preferably, Type I diabetes. The use includes, administering to a mammal in need of such treatment an effective amount of heregulin; Optionally, the methods of treatment comprise exposing mature beta cells or beta precursor cells to an effective amount of ErbB receptor ligand *ex vivo.* The cells treated *ex vivo* may then be administered to the mammal using suitable transplantation techniques.

In another embodiment, the invention provides methods of inducing or stimulating proliferation of beta precursor cells or mature beta cells. The methods include exposing beta precursor cells or mature beta cells to an effective amount of heregulin.

The invention further provides methods of inducing or stimulating beta precursor cell differentiation. In the methods, beta precursor cells or undifferentiated tissues containing such precursor cells are exposed to an effective amount of heregulin.

The invention also provides a composition comprising heregulin, betacellulin and a carrier. Preferably, the carrier is a pharmaceutically acceptable carrier. Methods of preparing such compositions are provided, and include admixing an effective amount of the ErbB receptor ligand with the carrier.

### Brief Description of the Drawings

Figure 1 shows the results of the assay described in Example 1 examining the effect of the ErbB receptor ligands. HB-EGF ("rh.HB-EGF"), heregulin ("rh.HRG"), amphiregulin ("rh.AR"), EGF ("rh.EGF"). TGF-alpha ("rh.TGF-a"), and betacellulin ("rh.BTC"), on expression of various markers (identified in the figure) in cultured primary murine fetal pancreatic cells.
Figure 2 shows a bar diagram illustrating the effect (measured as fold change of expression) of HB-EGF on marker expression - RPL19, NeuroD, Pax4, PDX-1, insulin. Glut2, GLK. Pax6. Glucagon, ISL1, Amylase, Somatostatin, and Cytoker 19.

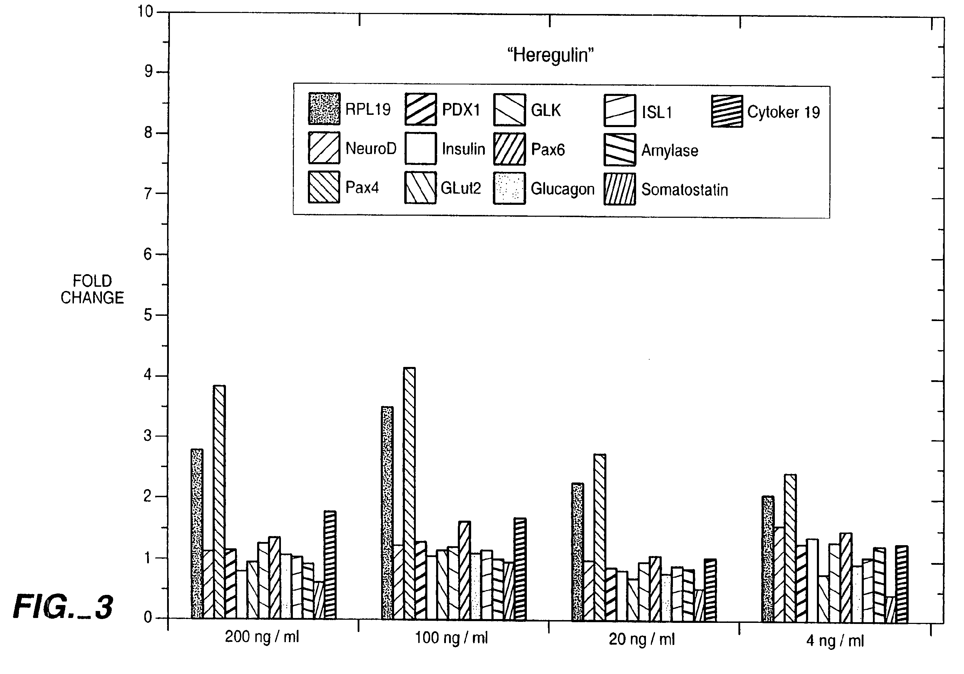

Figure 3 shows a bar diagram illustrating the effect (measured as fold change of expression) of heregulin on marker expression - RPL19, NeuroD, Pax4, PDX-1, insulin, Glut2, GLK. Pax6, Glucagon, ISL1. Amylase, Somatostatin, and Cytoker 19.

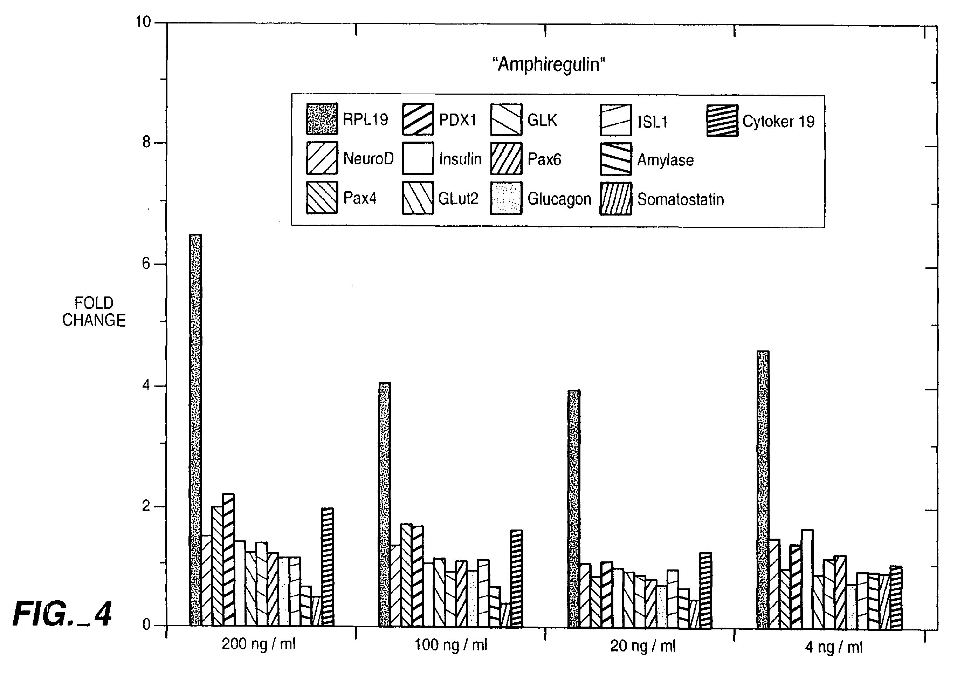

Figure 4 shows a bar diagram illustrating the effect (measured as fold change of expression) of amphiregulin on marker expression - RPL19. NeuroD, Pax4. PDX-1. insulin. Glut2, GLK. Pax6, Glucagon. ISL1, Amylase, Somatostatin, and Cytoker 19.

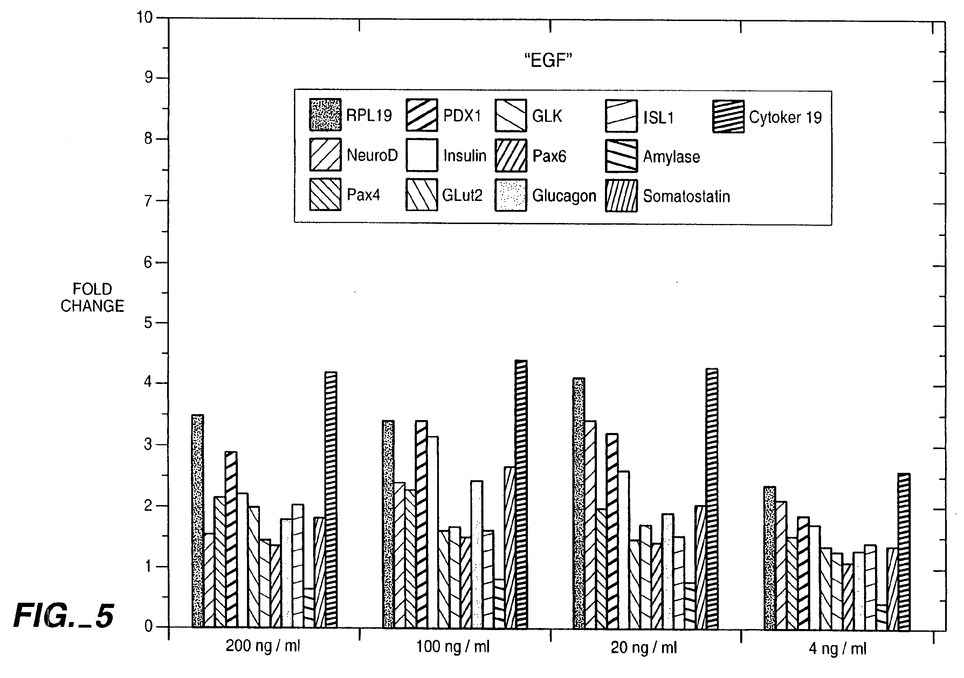

Figure 5 shows a bar diagram illustrating the effect (measured as fold change of expression) of EGF on marker expression - RPL19, NeuroD. Pax4. PDX-1, insulin, Glut2, GLK. Pax6, Glucagon. ISLI. Amylase, Somatostatin, and Cytoker 19.

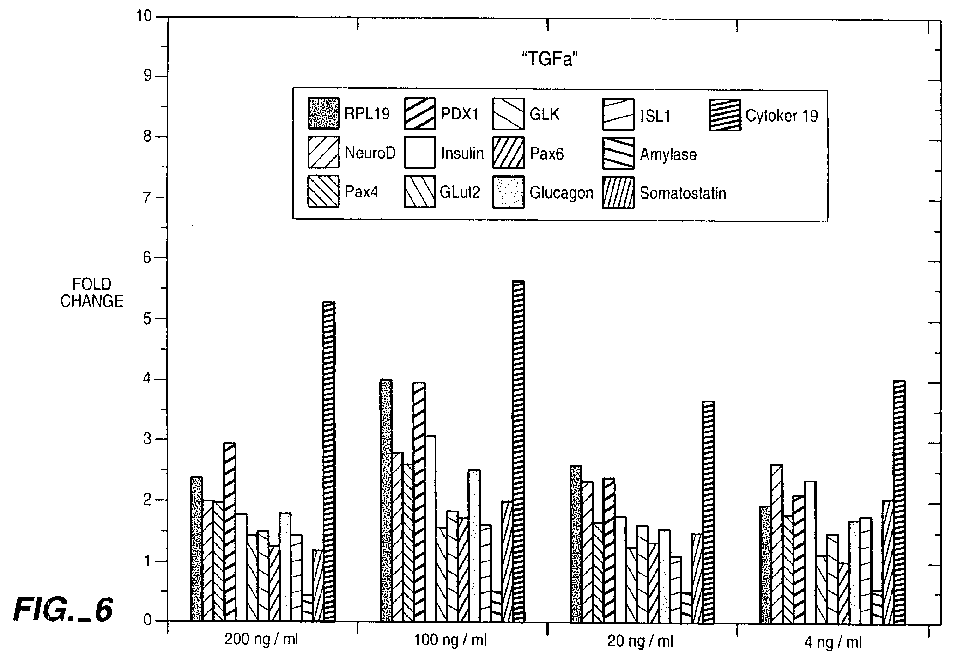

Figure 6 shows a bar diagram illustrating the effect (measured as fold change of expression) of TGF-alpha on marker expression - RPL19, NeuroD. Pax4, PDX-1. insulin, Glut2, GLK. Pax6. Glucagon, ISL1, Amylase, Somatostatin, and Cytoker 19.

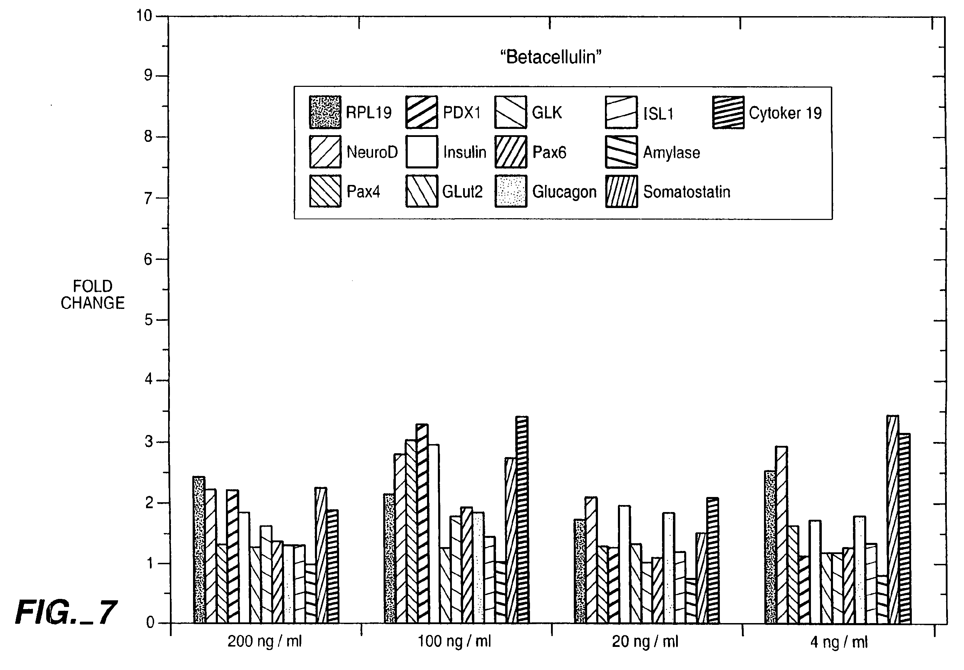

Figure 7 shows a bar diagram illustrating the effect (measured as fold change of expression) of betacellulin on marker expression - RPL 19. NeuroD. Pax4. PDX-1. insulin, Glut2. GLK. Pax6, Glucagon. ISL1. Amylase, Somatostatin, and Cytoker 19.
Figure 8 shows representative sections (at 20X) through pancreatic tissue from an untreated wild type animal (a), and a wild type (b), heregulin (+/-) (c). ErbB2 (+/-) (d), and ErbB3 (+/-) animal (e) that received heregulin treatment. The sections are from animals that received heregulin treatment for 14 days except in the case of the heregulin (-/-) treated animal which was dosed only for 5-6 days. The presence of ductal hyperplasia (shown by arrows) was most evident in the wild type and ErbB2 and ErbB3 (+/-) animal groups, possibly reflective of the longer exposure to heregulin.

### Detailed Description of the Invention

### I.Definitions

The term "ErbB receptor" as used herein refers to a receptor protein kinase which belongs to the ErbB receptor family and typically, in its native sequence form, comprises an extracellular domain, which may bind to one or more ErbB ligands (defined below), a lipophilic transmembrane domain, an intracellular tyrosine kinase domain, and a carboxyl-terminal signaling domain having one or more tyrosine residues which can be phosphorylated. The term "ErbB receptor" includes native sequence polypeptide receptors and amino acid sequence variants thereof. The ErbB receptor may be isolated from a variety of sources, such as from human tissue types or from another source, or prepared by recombinant and/or synthetic methods. ErbB receptors contemplated by the invention include but are not limited to, the EGFR. ErbB2. ErbB3. and ErbB4 receptors.

"ErbB1" and "EGFR" refer to the receptor disclosed in, for instance, Carpenter et al., Ann. Rev. Biochem. 56:881-914 (1987), including naturally occurring mutant forms thereof, such as the deletion mutant described in Humphrey et al., Proc. Natl. Acad. Sci. 87:4207-4211 (1990). *Erb*B1 refers to the gene encoding the ErbB I protein.

"ErbB2" and "HER2" refer to the receptor described, for instance, in Semba et al., Proc. Natl. Acad. Sci. 82:6497-6501 (1985) and Yamamoto et al., Nature 319:230-234 (1986) (GenBank accession number X03363). The term *erb*B2 refers to the gene encoding human ErbB2 and *neu* refers to the gene encoding rat p 185*^{neu}*.

"ErbB3" and "HER3" refer to the receptor as disclosed, for instance, in US Patents 5.183.884 and 5,480,968, as well as Kraus et al., Proc. Natl. Acad. Sci. 86:9193-9197 (1989).

"ErbB4" and "HER4" refer to the receptor as disclosed, for instance, in EP Patent Application 599,274, Plowman et al.. Proc. Natl. Acad. Sci. 90:1746-1750 (1993), and Plowman et al., Nature 366:473-475 (1993).

An "ErbB heterodimer" as referred to herein means a noncovalently associated oligomer comprising at least two different ErbB receptors. Such complexes may form when a cell expressing the two receptors is exposed to ErbB ligand(s) and can be isolated by immunoprecipitation and analyzed by SDS-PAGE as described in Sliwkowski et al.. J. Biol. Chem. 269:14661-14665 (1994). Examples of such heterodimers include EGFR-ErbB2. ErbB2-ErbB3. and ErbB3-ErbB4 complexes.

The terms "ErbB receptor ligand" and "ErbB ligand" refer to a polypeptide which binds to and/or activates one or more ErbB receptors. The term "ErbB ligand" includes native sequence polypeptide ligands and amino acid sequence variants thereof. The ErbB ligand may be isolated from a variety of sources, such as from human tissue types or from another source, or prepared by recombinant and/or synthetic methods. Preferably, for use in the methods disclosed herein, the ErbB ligand is prepared by recombinant methods. Binding of a candidate ErbB ligand to one or more ErbB receptors can be readily determined using known assays, such as those described in WO 98/35036 published August 13, 1998. Activation of an ErbB receptor refers to signal transduction (e.g.. that caused by an intracellular kinase domain of an ErbB receptor phosphorylating tyrosine residues in the ErbB receptor or a substrate polypeptide), mediated by ErbB ligand binding to an ErbB heterodimer comprising the ErbB receptor of interest. Generally, this will involve binding of an ErbB ligand to an ErbB heterodimer which activates a kinase domain of one or more of the ErbB receptors in the heterodimer and thereby results in phosphorylation of tyrosine residues in one or more of the receptors, and/or phosphorylation of tyrosine residues in additional substrate polypeptide(s). ErbB receptor phosphorylation can be quantified using various tyrosine phosphorylation assays, including those described in WO 98/35036 published August 13. 1998.

ErbB ligands contemplated by the invention include the polypeptides referred to below and described in, for example, the following respective journals and patents:
betacellulin [Shing et al.. Science 259:1604-1607 (1993); Sasada et al., Biochem. Biophys. Res. Commun. 190:1173 (1993)]:
heregulin (HRG), an ErbB ligand polypeptide encoded by the heregulin gene product as disclosed in US Patent 5.641.689 or Marchionni et al., Nature 362:312-318 (1993). Included within the scope of HRG as that term is used herein are heregulin-alpha, heregulin-betal, heregulin-beta2, and heregulin-beta3 [Holmes et al., Science 256:1205-1210 (1992): US Patent 5.641.869]; NDF [Peles et al., Cell 69:205-216 (1992)]; ARIA [Falls et al., Cell 72:801-815 (1993)]: GGF growth factor proteins [Marchionni et al., Nature 362:312-318 (1993); SMDF [Ho et al., J. Biol. Chem. 270:14523-14532 (1995); and gamma-heregulin [Schaefer et al., Oncogene 15:1385-1394 (1997)].

A "native sequence" polypeptide refers to a polypeptide having the same amino acid sequence as the polypeptide derived from nature. Such native sequence polypeptide may be isolated from nature or can be produced by recombinant and/or synthetic means. The term "native sequence" specifically encompasses naturally-occurring truncated or secreted forms (e.g., an extracellular domain sequence), naturally-occurring variant forms (e.g., alternatively spliced forms) and naturally-occurring allelic variants.

An "ErbB ligand variant" or "ErbB receptor ligand variant" refers to a ligand polypeptide other than native sequence ErbB ligand which binds to and/or activates one or more ErbB receptors and has at least about 80% amino acid sequence identity with its respective native sequence polypeptide, more preferably at least 90%, and even more preferably at least 95% amino acid sequence identity. ErbB receptor ligand variants include fragments of the native sequence ligand and having a consecutive sequence of at least 5, 10, 15, 20. 25, 30 or 40 amino acid residues from the native ligand sequence: amino acid sequence variants wherein an amino acid residue has been inserted N- or C-terminal to, or within, the sequence or its fragment as defined above: and amino acid sequence variants wherein one or more residues have been substituted by another residue. ErbB ligand variants include those containing predetermined mutations by, e.g., site-directed or PCR mutagenesis, and derived from various animal species such as rabbit, rat, porcine, non-human primate, equine, murine, and ovine.

"Percent (%) amino acid sequence identity" with respect to the sequences identified herein is defined as the percentage of amino acid residues in a candidate sequence that are identical with the amino acid residues in the ErbB ligand polypeptide sequence, after aligning the sequences and introducing gaps, if necessary, to achieve the maximum percent sequence identity, and not considering any conservative substitutions as part of the sequence identity. Methods for performing sequence alignment and determining sequence identity are known to the skilled artisan, may be performed without undue experimentation, and calculations of % identity values may be obtained with definiteness. For instance, the alignment may be performed using available computer programs, such as WU-BLAST-2 [Altschul et al., Methods in Enzymology 266:460-480 (1996)] and Align 2 [authored by Genentech. Inc. and filed with the US Copyright Office on December 10, 1991]. One may optionally perform the alignment using set default parameters in the computer software programs.

"Isolated polypeptide" means a polypeptide, such as HRG, which has been identified and separate and/or recovered from a component of its natural environment. Contaminant components of its natural environment are materials which would interfere with diagnostic or therapeutic uses for the polypeptide, and may include proteins, hormones, and other substances. In preferred embodiments, the polypeptide will be purified (1) to greater than 95% by weight of protein as determined by the Lowry method or other validated protein determination method, and most preferably more than 99% by weight, (2) to a degree sufficient to obtain at least 15 residues of N-terminal or internal amino acid sequence by use of the best commercially available amino acid sequenator marketed on the filing date hereof, or (3) to homogeneity by SDS-PAGE using Coomassie blue or, preferably, silver stain. Isolated polypeptide includes polypeptide *in situ* within recombinant cells since at least one component of the polypeptide natural environment will not be present. Isolated polypeptide includes polypeptide from one species in a recombinant cell culture of another species since the polypeptide in such circumstances will be devoid of source polypeptides. Ordinarily, however, isolated polypeptide will be prepared by at least one purification step.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxyl terminus of the heavy chain CH1 domain including one or more cysteine(s) from the antibody hinge region. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD. IgE, IgG, and IgM, and several of these may be further divided into subclasses (isotypes), e.g., IgG 1, IgG2, IgG3, IgG4, IgA, and IgA2. The subunit structures and three-dimensional configurations of different classes of immunoglobulins are well known.

The term "antibody" herein is used in the broadest sense and specifically covers monoclonal antibodies (including full length monoclonal antibodies), polyclonal antibodies, multispecific antibodies (e.g., bispecific antibodies), and antibody fragments so long as they exhibit the desired agonistic activity discussed in the present application.

"Antibody fragments" comprise a portion of a full length antibody, generally the antigen binding or variable domain thereof. Examples of antibody fragments include Fab. Fab'. F(ab')₂, and Fv fragments: diabodies: linear antibodies: single-chain antibody molecules: and multispecific antibodies formed from antibody fragments.

The term "monoclonal antibody" as used herein refers to an antibody obtained from a population of substantially homogeneous antibodies, i.e.. the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see. e.g., U.S. Patent No. 4,816.567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired agonistic activity [U.S. Patent No. 4.816.567: and Morrison et al., Proc. Natl. Acad. Sci. USA 81:6851-6855 (1984)].

"Humanized" forms of non-human (e.g., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or non-human primate having the desired specificity, affinity, and capacity. In some instances, framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986): Reichmann et al., Nature 332:323-329 (1988); and Presta. Curr, Op. Struct. Biol. 2:593-596 (1992).

"Single-chain Fv" or "sFv" antibody fragments comprise the V_{H} and V_{L} domains ofantibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv, see Pluckthun in the The Pharmacology of Monoclonal Antibodies, vol. 113, Rosenburg and Moore eds. Springer-Verlag. New York. pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404.097: WO 93/11161: and Hollinger et al., Proc. Natl. Acad. Sci. USA 90:6444-6448 (1993).

The expression linear antibodies, when used throughout this application, refers to the antibodies described in Zapata et al. Protein Eng. 8(10):1057-1062 (1995). Briefly, these antibodies comprise a pair of tandem Fv segments (V_{H}-C_{H}1-V_{H}-C_{H}1) which form a pair of antigen binding regions. Linear antibodies can be bispecific or monospecific.

"ErbB receptor agonist antibody" as used herein refers to an antibody against one or more ErbB receptors which binds to and/or activates one or more ErbB receptors. Binding and/or activation of an ErbB receptor may be determined using known assays, such as described herein.

The term "mature beta cell" refers to a differentiated epithelial cell which, in a normal physiological state, is capable of responding to changes in glucose concentration (between about 2 mM and 20 mM) by secreting insulin. The mature beta cell may further be characterized by the expression of insulin, glucokinase and/or PDX-1.

The term "beta precursor cell" as used herein refers to an epithelial cell capable of division and differentiation into a mature beta cell. The beta precursor cell may further be characterized by the expression of the gene markers PDX-1 and/or Pax4 and lack of expression of gene markers of non-epithelial origin (such as vimentin).

The terms "treating", "therapy" and "treatment" are used in the broadest sense and include prevention (prophylaxis), moderation, reduction and curing of the conditions described herein. "An effective amount" refers to that amount of ErbB ligand or ErbB receptor agonist antibody which stimulates or induces proliferation of mature beta cells or beta precursor cells *in vitro* and/or *in vivo*, or stimulates or induces beta precursor cell differentiation *in vitro* and/or *in vivo*.

Administration "in combination with" one or more further therapeutic agents includes simultaneous (concurrent) and consecutive administration in any order.

The term "pancreatic dysfunction" refers generally to condition(s) in mammals occurring as a result of a reduction or loss of beta cell function or by a reduction or loss of beta cell mass. The pancreatic dysfunction may be more particularly characterized, for example, by deficient levels of insulin in the mammal, deficient means of secreting insulin in the mammal or as a metabolic syndrome. The pancreatic dysfunction may be due, for instance, to insufficient differentiation of beta precursor cells into mature beta cells or destruction of beta cells that can occur in. e.g., insulitis or autoimmune disease.

The terms "diabetes" and "diabetes mellitus" are use in a broad sense and refer generally to the condition or syndrome in mammals associated with insulin deficiency, and include the conditions known in the art as insulin-dependent diabetes (also referred to as Type I diabetes or IDDM), noninsulin-dependent diabetes (also referred to as Type II diabetes or NIDDM), gestational diabetes, malnutrition-related diabetes (MRD) and maturity-onset diabetes of the young (also referred to as MODY).

The term "mammal" refers to any animal classified as a mammal, including humans, domestic and farm animals, dogs, horses, cats, etc. Preferably, the mammal is human.

### II. Methods and Compositions of the Invention

Applicants have found that various ErbB receptor ligands effectively alter expression of pancreatic cell transcription factors or markers, described in further detail below. Generally, precursor (or relatively undifferentiated) cells are identified by the presence or absence of specific cell markers and functionally by their respective ability to differentiate into the appropriate cell type. Marker definition for beta precursor cells is derived, at least in part, by a histological characterization of their origin and from transcription factors that are needed for pancreatic formation. Edlund, Diabetes 47:1817-1823 (1998): Bouwens, J. Pathology 184:234-239 (1998) and Sander et al., J. Mol. Med. 75:327-340 (1997) provide reviews of pancreatic tissue development and discussions of pancreatic cell markers, particularly expression of the various transcription factor markers and the role of such markers as indicia of stages of pancreatic cell development and function.

It is presently believed that at least a subset of ductular epithelial cells in mammals is capable of giving rise to functional endocrine cells (in both the fetal pancreas during embryogenesis and during adult life), and thus, the precursors would reasonably be expected to express, for instance, cytokeratin-19. a marker associated with pancreatic ductal epithelial cells.

The transcription factor. PDX-1. is typically expressed in a subset of gut endodermal cells in mammals and the temporal and spatial appearance of these cells is thought to be consistent with such cells being early pancreatic precursor cells. Also, genetic inactivation of PDX-1 may lead to the absence of a pancreas or pancreatic tissue in mammals. Accordingly, a beta precursor cell would reasonably be expected to express PDX-1. Absence of the transcription factors Pax4, Pax6 and/or NeuroD may lead to the absence of mature beta cells and mature islets, and so likewise, beta precursor cells may reasonably be expected to express one or all of these markers.

Insulin, glucose transporter 2 (glut2), the transcription factor ISL1, and glucokinase (GLK) are expressed in mature beta cells. They may also be expressed at a lower level in the beta precursor cells as such cells become committed to differentiation toward the mature beta cell phenotype. In the mature islet, each endocrine cell type generally expresses only one of the major hormones - the beta cells express insulin, the alpha cells express glucagon, and the delta cells express somatostatin. In contrast, an islet precursor cell may express the genes encoding more than one islet hormone. Accordingly, the beta precursor cells may also express glucagon and somatostatin. The gene encoding the ribosomal protein. RPL19 is expressed in a majority of cell types and can be used, such as described in the Examples, to represent changes in cell number.

It is believed that use of ErbB receptor ligands or ErbB receptor agonist antibodies to induce proliferation or growth of mature beta cells will be beneficial to increase insulin secretion in the mammal. Expansion of beta cell mass may constitute an important means to compensate for loss or dysfunction of beta cells occurring, for example, in diabetes. As beta precursor cells also appear to be present throughout childhood and adult life in mammals, it is further believed that use of ErbB receptor ligands or ErbB receptor agonist antibodies to induce or stimulate differentiation of such precursor cells into mature beta cells will be useful in treating conditions associated with insulin deficiency.

### A. Preparation of ErbB Ligands

The ErbB ligand (as well as ErbB receptor, which can be used for instance as an immunogen to prepare agonist antibodies) may be prepared by various techniques known in the art, including *in vitro* polypeptide synthetic methods or in recombinant cell culture using host-vector systems such as described below.

Optionally, mammalian host cells will be employed, and such hosts may or may not contain post-translational systems for processing ErbB ligand preprosequences in the normal fashion. If the host cells contain such systems, then it will be possible to recover natural subdomain fragments from the cultures. If not, then the proper processing can be accomplished by transforming the hosts with the required enzyme(s) or by supplying them in an *in vitro* method. However, it is not necessary to transform cells with the complete prepro or structural genes for a selected polypeptide when it is desired to only produce fragments of the sequences. For example, a start codon can be ligated to the 5' end of DNA encoding a ErbB ligand polypeptide, this DNA is used to transform host cells and the product expressed directly as the Met N-terminal form (if desired, the extraneous Met may be removed *in vitro* or by endogenous N-terminal demethionylases). Alternatively, ErbB ligand can be expressed as a fusion with a signal sequence recognized by the host cell, which will process and secrete the mature ErbB ligand as is further described below. Amino acid sequence variants of native sequence ErbB ligand can be produced in the same way.

### 1. Isolation of DNA

The DNA encoding ErbB ligand may be obtained from any cDNA library prepared from tissue believed to possess ErbB ligand mRNA and to express it at a detectable level. An ErbB ligand gene thus may be obtained from a genomic library.

Libraries can be screened with probes designed to identify the gene of interest or the protein encoded by it. For cDNA expression libraries, suitable probes include monoclonal or polyclonal antibodies that recognize and specifically bind to the ligand; oligonucleotides of about 20-80 bases in length that encode known or suspected portion of ErbB ligand cDNA from the same or different species; and/or complementary or homologous cDNAs or fragments thereof that encode the same or a similar gene. Appropriate probes for screening genomic DNA libraries include, but are not limited to, oligonucleotides; cDNAs or fragments thereof that encode the same or a similar gene; and/or homologous genomic DNAs or fragments thereof. Screening the cDNA or genomic library with the selected probe may be conducted using standard procedures as described in chapters 10-12 of Sambrook et al., Molecular- Cloning: A Laboratory Manual. (New York: Cold Spring Harbor Laboratory Press (1989).

An alternative means to isolate the gene encoding ErbB ligand is to use polymerase chain reaction (PCR) methodology, as described in section 14 of Sambrook et al.. *supra.* This method requires the use of oligonucleotide probes that will hybridize to ErbB ligand. Strategies for selection of oligonucleotides are described below.

Another alternative method for obtaining the gene of interest is to chemically synthesize it using one of the methods described in Engels et al. Agnew Chem. lnt. Ed. Engl. 28:216-734(1989). These methods include triester, phosphite, phosphoramidite and H-Phosphonate methods. PCR and other autoprimer methods, and oligonucleotide syntheses on solid supports. These methods may be used if the entire nucleic acid sequence of the gene is known, or the sequence of the nucleic acid complementary to the coding strand is available, or alternatively, if the target amino acid sequence is known, one may infer potential nucleic acid sequences using known and preferred coding residues for each amino acid residue.

An optional method is to use carefully selected oligonucleotide sequences to screen cDNA libraries from various tissues. The oligonucleotide sequences selected as probes should be of sufficient length and sufficiently unambiguous that false positives are minimized. The actual nucleotide sequence(s) may, for example, be based on conserved or highly homologous nucleotide sequences or regions of ErbB ligand. The oligonucleotides may be degenerate at one or more positions. The use of degenerate oligonucleotides may be of particular importance where a library is screened from a species in which preferential codon usage in that species is not known. The oligonucleotide must be labeled such that it can be detected upon hybridization to DNA in the library being screened. The preferred method of labeling is to use ³²P-labeled ATP with polynucleotide kinase, as is well known in the art, to radiolabel the oligonucleotide. However, other methods may be used to label the oligonucleotide, including, but not limited to, biotinylation or enzyme labeling.

### 2.Amino Acid Sequence Variants

Amino acid sequence variants ofErbB receptor ligands can be prepared by introducing appropriate nucleotide changes into the DNA, or by in *vitro* synthesis of the desired ligand polypeptide. Such variants include, for example, deletions from, or insertions or substitutions of, residues within the amino acid sequences of the native sequence ErbB ligand. Any combination of deletion, insertion, and substitution can be made to arrive at the final construct, provided that the final construct possesses the desired characteristics. The amino acid changes also may alter post-translational processes, such as changing the number of position of glycosylation sites, altering the membrane anchoring characteristics, altering the intra-cellular location of the polypeptide by inserting, deleting, or otherwise affecting the leader sequence of the native sequence polypeptide, or modifying its susceptibility to proteolytic cleavage.

In designing amino acid sequence variants of an ErbB ligand, the location of the mutation site and the nature of the mutation will depend on the polypeptide characteristic(s) to be modified. The sites for mutation can be modified individually or in series, e.g., by (1) substituting first with conservative amino acid choices and then with more radical selections depending upon the results achieved. (2) deleting the target residue, or (3) inserting residues of other receptor ligands adjacent to the located site.

A useful method for identification of certain residues or regions of the polypeptide that are preferred locations for mutagenesis is called "alanine scanning mutagenesis" as described by Cunningham and Wells (Science, 244:1081-1085. 1989). Here, a residue or group of target residues are identified (e.g.. charged residues such as arg, asp, his, lys, and glu) and replaced by a neutral or negatively charged amino acid (most preferably alanine or polyalanine) to affect the interaction of the amino acids with the surrounding aqueous environment in or outside the cell. Those domains demonstrating functional sensitivity to the substitutions then are refined by introducing further or other variants at or for the sites of substitution. Thus, while the site for introducing an amino acid sequence variation is predetermined, the nature of the mutation *per se* need not be predetermined. For example, to optimize the performance of a mutation at a given site, ala scanning or random mutagenesis may be conducted at the target codon or region and the expressed ErbB ligand variants are screened for the optimal combination of desired activity.

There are two principal variables in the construction of amino acid sequence variants: the location of the mutation site and the nature of the mutation. These are variants from the native sequence, and may represent naturally occurring alleles or predetermined mutant forms made by mutating the DNA, either to arrive at an allele or a variant not found in nature. In general, the location and nature of the mutation chosen will depend upon the characteristic to be modified.

Amino acid sequence deletions generally range from about 1 to 30 residues, more preferably about 1 to 10 residues, and typically about 1 to 5 are contiguous. The number of consecutive deletions may be selected so as to preserve the tertiary structure of the polypeptide in the affected domain. e.g., cysteine cross-linking, beta-pleated sheet or alpha helix.

Amino acid sequence insertions include amino- and/or carboxyl-terminal fusions ranging in length from one residue to polypeptides containing a hundred or more residues, as well as intrasequence insertions of single or multiple amino acid residues. Intrasequence insertions may range generally from about 1 to 10 residues, more preferably 1 to 5. and most preferably 1 to 3. Examples of terminal insertions include ErbB ligand with an N-terminal methionyl residue (an artifact of the direct expression of ErbB in bacterial recombinant cell culture), and fusion of a heterologous N-terminal signal sequence to the N-terminus of ErbB ligand to facilitate the secretion of mature ErbB ligand from recombinant host cells. Such signal sequences generally will be obtained from, and thus be homologous to, the intended host cell species. Suitable sequences include STII. tPA or lpp for *E.coli.* alpha factor for yeast, and viral signals such as herpes gD for mammalian cells.

Other insertional variants of ErbB ligand include the fusion to the N- or C-terminus of ErbB ligand of an immunogenic polypeptide. e.g., bacterial polypeptides such as beta-lactamase or an enzyme encoded by the *E.coli trp* locus, or yeast protein, bovine serum albumin. and chemotactic polypeptides. C-terminal fusions ofErbB ligand ECD with proteins having a long half-life such as immunoglobulin constant regions (or other immunoglobulin regions), albumin. or ferritin, as described in WO 89/02922. published 6 April 1989 are contemplated.

Another group of variants are amino acid substitution variants. These variants have at least one amino acid residue in the polypeptide removed and a different residue inserted in its place. The sites of greatest interest for substitutional mutagenesis include sites identified as the active site(s) of the polypeptide, and sites where the amino acids found in ErbB ligands from various species are substantially different in terms of side-chain bulk, charge, and/or hydrophobicity.

Non-conservative substitutions will entail exchanging a member of one of these classes for another. Such substituted residues may be introduced into regions of the polypeptide that are homologous with other receptor ligands, or, more preferably, into the non-homologous regions of the molecule.

In one embodiment, any methionyl residue other than the starting methionyl residue of the signal sequence, or any residue located within about three residues N- or C-terminal to each such methionyl residue, is substituted by another residue or deleted. Alternatively, about 1-3 residues are inserted adjacent to such sites.

Any cysteine residues not involved in maintaining the proper conformation of ErbB ligand also may be substituted, generally with serine, to improve the oxidative stability of the molecule and prevent aberrant cross-linking.

DNA encoding amino acid sequence variants of the ErbB ligand can be prepared by a variety of methods known in the art. These methods include, but are not limited to, isolation from a natural source (in the case of naturally occurring amino acid sequence variants) orpreparation by oligonucleotide-mediated (site-directed) mutagenesis, PCR mutagenesis, and cassette mutagenesis of an earlier prepared variant or a non-variant version. These techniques may utilize nucleic acid (DNA or RNA), or nucleic acid complementary to such nucleic acid.

Oligonucleotide-mediated mutagenesis is an optional method for preparing substitution, deletion, and insertion variants. This technique is known in the art as described by Adelman et al., DNA. 2:183 (1983). Briefly, DNA is altered by hybridizing an oligonucleotide encoding the desired mutation to a DNA template, where the template is the single-stranded form of a plasmid.or bacteriophage containing the unaltered or native DNA sequence. After hybridization, a DNA polymerase is used to synthesize an entire second complementary strand of the template that will thus incorporate the oligonucleotide primer, and will code for the selected alteration in the DNA.

Generally, oligonucleotides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridize properly to the single-stranded DNA template molecule. The oligonucleotides are readily synthesized using techniques known in the art such as that described by Crea et al.. Proc. Natl. Acad. Sci.. 75:L5765 (1978).

Single-stranded DNA template may also be generated by denaturing double-stranded plasmid (or other) DNA using standard techniques.

For alteration of the native DNA sequence (to generate amino acid sequence variants, for example), the oligonucleotide is hybridized to the single-stranded template under suitable hybridization conditions. A DNA polymerizing enzyme, usually the Klenow fragment of DNA polymerase I. is then added to synthesize the complementary strand of the template using the oligonucleotide as a primer for synthesis. A heteroduplex molecule is thus formed such that one strand of DNA encodes the mutated form of the polypeptide, and the other strand (the original template) encodes the native, unaltered sequence of the polypeptide. This heteroduplex molecule is then transformed into a suitable host cell, usually a prokaryote such as *E.coli* JM 101. After the cells are grown, they are plated onto agarose plates and screened using the oligonucleotide primer radiolabeled with ³²P-phosphate to identify the bacterial colonies that contain the mutated DNA. The mutated region is then removed and placed in an appropriate vector for protein production, generally an expression vector of the type typically employed for transformation of an appropriate host.

The method described immediately above may be modified such that a homoduplex molecule is created wherein both strands of the plasmid contain the mutation(s). The modifications are as follows: the single-stranded oligonucleotide is annealed to the single-stranded template, as described above. A mixture of three deoxyribonucleotides, deoxyriboadenosine (dATP), deoxyriboguanosine (dGTD), and deoxyribothymidine (dTTP), is combined with a modified thio-deoxyribocytosine called dCTP-(aS) (which can be obtained from Amersham Corporation). This mixture is added to the template-oligonucleotide complex. Upon addition of DNA polymerase to this mixture, a strand of DNA identical to the template except for the mutated bases is generated. In addition, this new strand of DNA will contain dCTP-(aS) instead of dCTP, which serves to protect it from restriction endonuclease digestion. After the template strand of the double-stranded heteroduplex is nicked with an appropriate restriction enzyme, the template strand can be digested with *Exo*III nuclease or another appropriate nuclease past the region that contains the site(s) to be mutagenized. The reaction is then stopped to leave a molecule that is only partially single-stranded. A complete double-stranded DNA homoduplex is then formed using DNA polymerase in the presence of all four deoxyribonucleotide triphosphates, ATP, and DNA ligase. This homoduplex molecule can then be transformed into a suitable host cell such as *E.coli* JM101, as described above.

DNA encoding variants with more than one amino acid to be substituted may be generated in one of several ways. If the amino acids are located close together in the polypeptide chain, they may be mutated simultaneously using one oligonucleotide that codes for all of the desired amino acid substitutions. If, however, the amino acids are located some distance from each other (separated by more than about ten amino acids), it is more difficult to generate a single oligonucleotide that encodes all of the desired changes. Instead, one of two alternative methods may be employed.

In the first method, a separate oligonucleotide is generated for each amino acid to be substituted. The oligonucleotides are then annealed to the single-stranded template DNA simultaneously, and the second strand of DNA that is synthesized from the template will encode all of the desired amino acid substitutions.

The alternative method involves two or more rounds of mutagenesis to produce the desired mutant. The first round is as described for the single mutants: wild-type DNA is used for the template, an oligonucleotide encoding the first desired amino acid substitution(s) is annealed to this template, and the heteroduplex DNA molecule is then generated. The second round of mutagenesis utilizes the mutated DNA produced in the first round of mutagenesis as the template. Thus, this template already contains one or more mutations. The oligonucleotide encoding the additional desired amino acid substitution(s) is then annealed to this template, and the resulting strand of DNA now encodes mutations from both the first and second rounds of mutagenesis. This resultant DNA can be used as a template in a third round of mutagenesis, and so on.

PCR mutagenesis is also suitable for making amino acid variants. While the following discussion refers to DNA. it is understood that the technique also finds application with RNA. The PCR technique generally refers to the following procedure (see Erlich. *supra,* the chapter by R. Higuchi. p. 61-70). When small amounts of template DNA are used as starting material in a PCR. primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA. one of the primers is designed to overlap the position of the mutation and to contain the mutation; the sequence of the other primer must be identical to a stretch of sequence of the opposite strand of the plasmid, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions.

If the ratio of template to product material is extremely low, the vast majority of product DNA fragments incorporate the desired mutation(s). This product material is used to replace the corresponding region in the plasmid that served as PCR template using standard DNA technology. Mutations at separate positions can be introduced simultaneously by either using a mutant second primer, or performing a second PCR with different mutant primers and ligating the two resulting PCR fragments simultaneously to the vector fragment in a three (or more)-part ligation.

Another method for preparing variants, cassette mutagenesis, is based on the technique described by Wells et al. (Gene. 34:315, 1985). The starting material is the plasmid (or other vector) comprising DNA to be mutated. The codon(s) in the DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the DNA. After the restriction sites have been introduced into the plasmid, the plasmid is cut at these sites to linearize it. A double-stranded oligonucleotide encoding the sequence of the DNA between the restriction sites, but containing the desired mutation(s), is synthesized using standard procedures. The two strands are synthesized separately and then hybridized together using standard techniques. This double-stranded oligonucleotide is referred to as the cassette. This cassette is designed to have 3' and 5' ends that are compatible with the ends of the linearized plasmid, such that it can be directly ligated to the plasmid.

Any such methods and techniques may be employed to prepare or identify ErbB ligand variants useful in the present invention. In particular, reference is made to WO 98/35036 which discloses various heregulin variants which may be useful in the present invention.

### 3. Insertion of DNA into a Cloning Vehicle

The cDNA or genomic DNA encoding the native or variant polypeptide is inserted into a replicable vector for further cloning (amplification of the DNA) or for expression. Many vectors are available, and selection of the appropriate vector will depend on 1) whether it is to be used for DNA amplification or for DNA expression, 2) the size of the DNA to be inserted into the vector, and 3) the host cell to be transformed with the vector. Each vector contains various components depending on its function (amplification of DNA or expression of DNA) and the host cell for which it is compatible. The vector components generally include, but are not limited to, one or more of the following: a signal sequence, an origin of replication, one or more marker genes, an enhancer element, a promoter, and a transcription termination sequence.

### (i) Signal Sequence Component

In general, the signal sequence may be a component of the vector, or it may be a part of the DNA that is inserted into the vector. The native DNA is believed to encode a signal sequence at the amino terminus (5' end of the DNA encoding the polypeptide) of the polypeptide that is cleaved during post-translational processing of the polypeptide. For instance, native ErbB ligand may be secreted from the cell, but remains lodged in the membrane because it contains a transmembrane domain and a cytoplasmic region in the carboxyl terminal region of the polypeptide. Thus, in a secreted, soluble version of ErbB ligand the carboxyl terminal domain of the molecule, including the transmembrane domain, is ordinarily deleted. This truncated variant ErbB ligand may be secreted from the cell, provided that the DNA encoding the truncated variant encodes a signal sequence recognized by the host.

The selected polypeptide may be expressed not only directly, but also as a fusion with a heterologous polypeptide, preferably a signal sequence or other polypeptide having a specific cleavage site at the N-and/or C-terminus of the mature polypeptide. In general, the signal sequence may be a component of the vector, or it may be a part of the DNA that is inserted into the vector. Included within the scope of this invention are ErbB ligands with the native signal sequence deleted and replaced with a heterologous signal sequence. The heterologous signal sequence selected should be one that is recognized and processed. i.e.. cleaved by a signal peptidase, by the host cell. For prokaryotic host cells that do not recognize and process the native ErbB ligand signal sequence, the signal sequence is substituted by a prokaryotic signal sequence selected, for example, from the group of the alkaline phosphatase, penicillinase, lpp, or heat-stable enterotoxin II leaders. For yeast secretion, the native ErbB ligand signal sequence may be substituted by the yeast invertase, alpha factor, or acid phosphatase leaders. In mammalian cell expression, the native signal sequence is satisfactory, although other mammalian signal sequences may be suitable.

### (ii) Origin of Replication Component

Both expression and cloning vectors generally contain a nucleic acid sequence that enables the vector to replicate in one or more selected host cells. Generally, in cloning vectors, this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2m plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Most expression vectors are "shuttle" vectors, i.e.. they are capable of replication in at least one class of organisms, but can be transfected into another organism for expression. For example, a vector is cloned in *E.coli* and then the same vector is transfected into yeast or mammalian cells for expression.

DNA may also be amplified by insertion into the host genome. This is readily accomplished using *Bacillus* species as hosts, for example, by including in the vector a DNA sequence that is complementary to a sequence found in *Bacillus* genomic DNA. Transfection of *Bacillus* with this vector results in homologous recombination with the genome and insertion of the DNA encoding the selected polypeptide. DNA can be amplified by PCR and directly transfected into the host cells without any replication component.

### (iii) Selection Gene Component

Expression and cloning vectors should contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g.. ampicillin, neomycin, methotrexate, or tetracycline. (b) complement auxotrophic deficiencies, or (c) supply critical nutrients not available from complex media, e.g.. the gene encoding D-alanine racemase for *Bacilli.*

One example of a selection scheme utilizes a drug to arrest growth of a host cell. Those cells that are successfully transformed with a heterologous gene express a protein conferring drug resistance and thus survive the selection regimen. Examples of such dominant selection use the drugs neomycin (Southern et al., J. Molec. Appl. Genet. 1:327, 1982), mycophenolic acid (Mulligan et al., Science 209:1422.1980) or hygromycin (Sugden et al., Mol. Cell. Biol. 5:410-413, 1985). The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up the nucleic acid, such as dihydrofolate reductase (DHFR) or thymidine kinase. The mammalian cell transformants are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes the selected polypeptide. Amplification is the process by which genes in greater demand for the production of a protein critical for growth are reiterated in tandem within the chromosomes of successive generations of recombinant cells.

For example, cells transformed with the DHFR selection gene are first identified by culturing all of the transformants in a culture medium that contains methotrexate (Mtx), a competitive antagonist of DHFR. An appropriate host cell, when wild-type DHFR is employed, is the Chinese hamster ovary (CHO) cell line deficient in DHFR activity, prepared and propagated as described by Urlaub and Chasin, Proc. Natl. Acad. Sci. USA, 77:4216, 1980. The transformed cells are then exposed to increased levels of methotrexate. This leads to the synthesis of multiple copies of the DHFR gene, and, concomitantly, multiple copies of other DNA comprising the expression vectors, such as the DNA encoding ErbB ligand. This amplification technique can be used with any otherwise suitable host, e.g., ATCC No. CCL61 CHO-K1, notwithstanding the presence of endogenous DHFR if for example, a mutant DHFR gene that is highly resistant to Mtx is employed (EP 117,060). Alternatively, host cells (particularly wild-type hosts that contain endogenous DHFR) transformed or co-transformed with DNA sequences encoding the selected polypeptide, wild-type DHFR protein, and another selectable marker such as aminoglycoside 3' phosphotransferase (APH) can be selected by cell growth in medium containing a selection agent for the selectable marker such as an aminoglycosidic antibiotic. e.g., kanamycin, neomycin, or G418 (see U.S. Patent No. 4,965,199).

A suitable selection gene for use in yeast is the *trp*1 gene present in the yeast plasmid Yrp7 (Stinchcomb et al., Nature, 282:39. 1979: Kingsman et al.. Gene, 7:141, 1979: or Tschemper et ai., Gene, 10: 157. 1980). The *trp*1 gene provides a selection marker for a mutant strain of yeast lacking the ability to grow in tryptophan, for example. ATCC No. 44076 or PEP4-1 (Jones, Genetics, 85:12. 1977). The presence of the *trp*1 lesion in the yeast host cell genome then provides an effective environment for detecting transformation by growth in the absence of tryptophan. Similarly, Leu2-deficient yeast strains (ATCC 20,622 or 38.626) are complemented by known plasmids bearing the *Leu2* gene.

### (iv) Promoter Component

Expression and cloning vectors usually contain a promoter that is recognized by the host organism and is operably linked to the nucleic acid encoding the polypeptide. Promoters are untranslated sequences located upstream (5') to the start codon of a structural gene (generally within about 100 to 1000 bp) that control the transcription and translation of a particular nucleic acid sequence, such as ErbB ligand to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to DNA encoding ErbB ligand by removing the promoter from the source DNA by restriction enzyme digestion and inserting the isolated promoter sequence into the vector. Both the native ErbB ligand promoter sequence and many heterologous promoters may be used to direct amplification and/or expression of ErbB ligand DNA. However, heterologous promoters are preferred, as they generally permit greater transcription and higher yields of expressed ErbB ligand as compared to the native ErbB ligand promoter.

Promoters suitable for use with prokaryotic hosts include the β-lactamase and lactose promoter systems (Chang et al.. Nature, 275: 615. 1978: and Goeddel et al., Nature 281: 544.1979), alkaline phosphatase, a tryptophan (trp) promoter system (Goeddel. Nucleic Acids Res., 8: 4057. 1980 and EP 36.776), tPA (U.S. 5,641.655) and hybrid promoters such as the tac promoter (deBoer et al.. Proc. Natl. Acad. Sci. USA 80: 21-25, 1983). However, other known bacterial promoters are suitable. Their nucleotide sequences have been published, thereby enabling a skilled worker operably to ligate them to DNA encoding the selected polypeptide (Siebenlist et al., Cell 20: 269, 1980) using linkers or adapters to supply any required restriction sites. Promoters for use in bacterial systems also generally will contain a Shine-Dalgarno (S.D.) sequence operably linked to the encoding DNA.

Suitable promoting sequences for use with yeast hosts include the promoters for 3-phosphoglycerate kinase (Hitzeman et al., J. Biol. Chem., 255: 2073. 1980) or other glycolytic enzymes (Hess et al., J. Adv. Enzyme Reg 7: 149, 1968: and Holland. Biochemistry 17: 4900, 1978), such as enolase glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in Hitzeman et al., EP 73.657A. Yeast enhancers also are advantageously used with yeast promoters.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into mammalian expression vectors.

Gene transcription from vectors in mammalian host cells may be controlled by promoters obtained from the genomes of viruses such as polyoma virus, fowlpox virus (UK 2,211.504, published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, and from the promoter normally associated with ErbB ligand sequence, provided such promoters are compatible with the host cell systems.

The early and late promoters of the SV40 virus are conveniently obtained as an SV40 restriction fragment that also contains the SV40 viral origin of replication (Fiers et al., Nature, 273:113 (1978); Mulligan and Berg, Science, 209: 14221427 (1980); Pavlakis et al., Proc. Natl..Acad. Sci. USA. 78: 73 98-7402 (1981). The immediate early promoter of the human cytomegalovirus is conveniently obtained as a *HindIII* restriction fragment (Greenaway et al., Gene, 18: 355-360 (1982)). A system for expressing DNA in mammalian hosts using the bovine papilloma virus as a vector is disclosed in U.S. Patent No.4,419,446. A modification of this system is described in U.S. Patent No. 4.601.978. See also Gray et al., Nature. 295: 503-508 (1982) on expressing cDNA encoding immune interferon in monkey cells: Reyes et al., Nature, 297; 598-601 (1982) on expression of human β-interferon cDNA in mouse cells under the control of a thymidine kinase promoter from herpes simplex virus: Canaani and Berg. Proc. Natl. Acad. Sci. USA, 79: 5166-5170 (1982) on expression of the human interferon gene in cultured mouse and rabbit cells; and Gorman et al.. Proc. Natl. Acad. Sci. USA. 79: 6777-6781 (1982) on expression of bacterial CAT sequences in CV-1 monkey kidney cells, chicken embryo fibroblasts. Chinese hamster ovary cells. HeLa cells, and mouse NIH-3T3 cells using the Rous sarcoma virus long terminal repeat as a promoter.

### (v) Enhancer Element Component

Transcription of a DNA encoding a selected polypeptide of this invention by higher eukaryotes is often increased by inserting an enhancer sequence into the vector. Enhancers are cis-acting elements of DNA. usually about from 10-300 bp. that act on a promoter to increase its transcription. Enhancers are relatively orientation and position independent having been found 5' (Laimins, et al., Proc. Natl. Acad. Sci. USA, 78: 993, 1981) and 3' (Lusky et al., Mol. Cell Bio.. 3: 1108, 1983) to the transcription unit, within an intron (Banerji et al., Cell, 33: 729. 1983) as well as within the coding sequence itself (Osborne et al.. Mol. Cell Bio.. 4: 1293, 1984). Many enhancer sequences are now known from mammalian genes (globin, elastase, albumin, α-feto protein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers (see also Yaniv, Nature, 297: 17-18(1982)) on enhancing elements for activation of eukaryotic promoters. The enhancer may be spliced into the vector at a position 5' or 3' to ErbB ligand DNA. but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain nucleotide segments transcribed as polyadenylated fragments in the untranslated portion of the mRNA encoding the polypeptide. The 3' untranslated regions also include transcription termination sites.

Construction of suitable vectors containing one or more of the above listed components and the desired coding and control sequences employs standard ligation techniques. Isolated plasmids or DNA fragments are cleaved, tailored, and relegated in the form desired to generate the plasmids required.

For analysis to confirm correct sequences in plasmids constructed, the ligation mixtures are used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing et al.. Nucleic Acids Res. 9 : 309 (1981) or by the method of Maxam et al., Methods in Enzymology 65: 499 (1980).

Particularly useful in the practice of this invention are expression vectors that provide for the transient expression in mammalian cells. In general, transient expression involves the use of an expression vector that is able to replicate efficiently in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying useful analogs and variants. Such a transient expression system is described in U.S. 5.024.939.

Other methods, vectors, and host cells suitable for adaptation to the synthesis of the selected polypeptide in recombinant vertebrate cell culture are described in Gething et al., Nature 293: 620-625, 1981; Mantei et al., Nature. 281: 40-46. 1979; Levinson et al., EP 117,060 and EP 117.058.

### 4. Selection and Transformation of Host Cells

Suitable host cells for cloning or expressing the vectors herein are the prokaryote, yeast, or higher eukaryote cells described above. Suitable prokaryotes include eubacteria, such as Gram-negative or Gram-positive organisms,. for example. *E. coli, Bacilli* such as *B. subtilis. Pseudomonas species* such as P. aeruginosa. Salmonella typhimurium, or Serratia marcescans. One preferred *E. coli* cloning host is *E. coli* 294 (ATCC 31.446), although other strains such as *E. coli* X1776 (ATCC 31,537), and *E. coli* W3110 (ATCC 27,325) are suitable. These examples are illustrative rather than limiting. Preferably the host cell should secrete minimal amounts of proteolytic enzymes. Alternatively, *in vitro* methods of cloning, e.g., PCR or other nucleic acid polymerase reactions, are suitable.

In addition to prokaryotes, eukaryotic microbes such as filamentous fungi or yeast are suitable hosts. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe* (Beach and Nurse, Nature, 290:140 (1981); EP 139,383, published May 2, 1985), Kluyveromyces hosts (U.S. Patent 4.943.529) such as. e.g., *K*. lactis (Louvencourt et al., J. Bacteriol., 737(1983); K. fragilis, K. bulgaricus, K. thermotolerans, and *K*. marxianus, yarrowia (EP 402,226); Pichia pastoris (EP 183,070), Sreekrishna et al., LT. Basic Microbiol., 28: 265-278 (1988); *Candida, Trichoderma reesia (*EP 244,234*);* Neurospora crassa (Case et al., Proc. Natl. Acad. Sci. USA, 76:5259-5263 (1979), and filamentous fungi such as, e.g, Neurospora, Penicillium, Tolypocladium (WO 91/00357, published 10 January 1991), and Aspergillus hosts such as A. nidulans (Ballance et al., Biochem. Biophys. Res. Commun., 112: 284-289 (1983); Tilburn et al., Gene, 26: 205-221 (1983); Yelton et al., Proc. Natl. Acad. Sci. USA, 81: 1470-1474 (1984) and A. Niger (Kelly and Hynes, EMBO J., 4: 475-479 (1985)).

Suitable host cells for the expression of glycosylated polypeptide are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito). *Aedes albopictus* (mosquito). *Drosophila melanogaster* (fruitfly), and *Bombyx mori* host cells have been identified (see. e.g.. Luckow et al.. Bio/Technology,, 6: 47-55 (1988); Miller et al., in Genetic Engineering, Set low. J.K. et al., eds., Vol. 8 (Plenum Publishing, 1986), pp. 277-279: and Maeda et al.. Nature, 315:592-594 (1985)). A variety of such viral strains are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells. Plant cell cultures of cottonn corn. potato, soybean, petunian tomato, and tobacco can be utilized as hosts. Typically, plant cells are transfected by incubation with certain strains of the bacterium *Agrobacterium tumefaciens.* During incubation of the plant cell culture with *A*. *tumefaciens,* the DNA encoding ErbB ligand is transferred to the plant cell host such that it is transfected, and will, under appropriate conditions, express ErbB ligand DNA. In addition, regulatory and signal sequences compatible with plant cells are available, such as the nopaline synthase promoter and polyadenylation signal sequences (Depicker et al., Mol. Appl. Gen., 1: 561 (1982)). In addition. DNA segments isolated from the upstream region of the T-DNA 780 gene are capable of activating or increasing transcription levels of plant-expressible genes in recombinant DNA-containing plant tissue (see EP 321.196, published 21 June 1989).

However, interest has been greatest in vertebrate cells, and propagation of vertebrate cells in culture (tissue culture) has become a routine procedure in recent years (Tissue Culture, Academic Press, Kruse and Patterson, editors (1973)). Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol., 36: 59. 1977): baby hamster kidney cells (BHK. ATCC CCL 10); Chinese hamster ovary cells/-DHFR (CHO. Urlaub and Chasin. Proc. Natl. Acad. Sci. USA. 77: 4216 (1980)): mouse sertoli cells (TM4, Mather. Biol. Reprod., 23: 243-251 (1980)); monkey kidney cells (CV 1 ATCC CCL 70); African green monkey kidney cells (VERO-76. ATCC CRL-1587): human cervical carcinoma cells (HELA, ATCC CCL 2); canine kidney cells (MDCK. ATCC CCL 34): buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N. Y. Acad. Sci., 383: 44-68 (1982)); MRC 5 cells; FS4 cells; and a human hepatoma cell line (Hep G2). Preferred host cells are human embryonic kidney 293 and Chinese hamster ovary cells.

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example. CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integration. Depending on the host cell used, transformation is done using standard techniques appropriate to such cells. The calcium treatment employing calcium chloride, as described in section 1.82 of Sambrook *et al., supra, is* generally used for prokaryotes or other cells that contain substantial cell-wall barriers. Infection with *Agrobacterium tumefaciens is* used for transformation of certain plant cells, as described by Shaw et al., Gene, 23: 315 (1983) and WO 89/05859, published 29 June 1989. For mammalian cells without such cell walls, the calcium phosphate precipitation method described in sections 16.30-16.37 of Sambrook *et al, supra, is* preferred. General aspects of mammalian cell host system transformations have been described by Axel in U.S. 4.399.216. issued 16 August 1983. Transformations into yeast are typically carried out according to the method of Van Solingen et al., J. Bact., 130: 946 (1977) and Hsiao et al., Proc. Natl. Acad. Sci. (USA), 76: 3829 (1979). However, other methods for introducing DNA into cells such as by nuclear injection, electroporation, or protoplast fusion may also be used.

### 5. Culturing the Host Cells

Prokaryotic cells used to produce the ErbB ligand are cultured in suitable media as described generally in Sambrook *et al., supra.*

The mammalian host cells used to produce the ErbB ligand may be cultured in a variety of media. Commercially available media such as Ham's F10 (Sigma). Minimal Essential 30 Medium ((MEM). Sigma), RPMI-1640 (Sigma), and Dulbecco's Modified Eagle's Medium ((DMEM), Sigma) are suitable for culturing the host cells. In addition, any of the media described in Ham and Wallace, Meth. Enz., 58: 44 (1979), Barnes and Sato, Anal. Biochem., 102: 255 (1980), U.S. 4,767,704; 4.657,866; 4,927.762: or 4.560,655; WO 90/03430; WO 87/00195; U.S. Patent No. Re. 30,985: U.S. 5,122,469, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as GENTAMYCIN drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH. and the like, are those previously used with the host cell selected for expression, and will be apparent to the ordinarily skilled artisan.

The host cells referred to in this disclosure encompass cells in in *vitro* culture as well as cells that are within a host animal.

It is further envisioned that the ErbB ligand may be produced by homologous recombination, or with recombinant production methods utilizing control elements introduced into cells already containing DNA encoding the polypeptide currently in use in the field. For example, a powerful promoter/enhancer element, a suppresser, or an exogenous transcription modulatory element is inserted in the genome of the intended host cell in proximity and orientation sufficient to influence the transcription of DNA encoding the desired polypeptide.

### 6. Detecting Gene Amplification/Expression

Gene amplification and/or expression may be measured in a sample directly, for example, by conventional Southern blotting. Northern blotting to quantitate the transcription of mRNA (Thomas, Proc. Natl. Acad. Sci. USA, 77:5201-5205 (1980)), dot blotting (DNA analysis), or *in situ* hybridization, using an appropriately labeled probe based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies which may be labeled with a wide variety of labels, such as radionuclides, fluorescers, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product. With immunohistochemical staining techniques, a cell sample is prepared, typically dehydration and fixation, followed by reaction with labeled antibodies specific for the gene product coupled where the labels are usually visually detectable such as enzymatic labels, fluorescent labels, luminescent labels, and the like. A particularly sensitive staining technique suitable for use in the present invention is described by Hsu et al., Am. J. Clin. Path., 75:734-738 (1980).

Antibodies useful for immunohistochemical staining and/or assay of sample fluids may be either monoclonal or polyclonal, and may be prepared in any mammal. Conveniently, the antibodies may be prepared against a native ErbB ligand or against a synthetic peptide based on the DNA sequences provided herein as described further below.

### 7. Purification of the Polypeptides

The ErbB ligand may be recovered from a cellular membrane fraction. Alternatively, a proteolytically cleaved or a truncated expressed soluble fragment or subdomain are recovered from the culture medium as a soluble polypeptide. The polypeptide can be recovered from host cell lysates when directly expressed without a secretory signal.

When ErbB ligand is expressed in a recombinant cell other than one of human origin. ErbB ligand is completely free of proteins or polypeptides of human origin. However, it is desirable to purify ErbB ligand from recombinant cell proteins or polypeptides to obtain preparations that are substantially homogeneous as to ErbB ligand. As a first step, the culture medium or lysate is centrifuged to remove particulate cell debris. The membrane and soluble protein fractions are then separated. ErbB ligand can then be purified from both the soluble protein fraction (requiring the presence of a protease) and from the membrane fraction of the culture lysate, depending on whether ErbB ligand is membrane bound. The following procedures are exemplary of suitable purification procedures: fractionation on immunoaffinity or ion-exchange columns; ethanol precipitation; reverse phase HPLC: chromatography on silica, heparin SEPHAROSE or on a cation exchange resin such as DEAE: chromatofocusing; SDS-PAGE: ammonium sulfate precipitation: and gel filtration using, for example. SEPHADEX G-75.

Polypeptide variants in which residues have been deleted, inserted or substituted are recovered in the same fashion as the native polypeptide, taking account of any substantial changes in properties occasioned by the variation. For example, preparation of an ErbB ligand fusion with another protein or polypeptide. e.g.. a bacterial or viral antigen, facilitates purification: an immunoaffinity column containing antibody to the antigen can be used to adsorb the fusion. Immunoaffinity columns such as a rabbit polyclonal anti-ErbB ligand column can be employed to absorb ErbB ligand variant by binding it to at least one remaining immune epitope. A protease inhibitor such as phenylmethylsulfonylfluoride (PMSF) also may be useful to inhibit proteolytic degradation during purification, and antibiotics may be included to prevent the growth of adventitious contaminants. One skilled in the art will appreciate that purification methods suitable for native polypeptide may require modification to account for changes in the character of variants or upon expression in recombinant cell culture.

### 8. Covalent Modifications of the Polypeptides

Covalent modifications of the ErbB ligands are included within the scope of this invention. Both native sequence and amino acid sequence variants optionally are covalently modified. One type of covalent modification included within the scope of this invention is an ErbB ligand fragment. ErbB ligand fragments, such as those having up to about 40 amino acid residues are conveniently prepared by chemical synthesis, or by enzymatic or chemical cleavage of the full-length ErbB ligand polypeptide or ErbB ligand variant polypeptide. Other types of covalent modifications of ErbB ligands are introduced into the molecule by reacting targeted amino acid residues of ErbB ligands with an organic derivatizing agent that is capable of reacting with selected side chains or the N- or C-terminal residues.

Cysteinyl residues most commonly are reacted with α-haloacetates (and corresponding amines) such as chloroacetic acid or chloroacetamide, to give carboxymethyl or carboxyamidomethyl derivatives. Cysteinyl residues also are derivatized by reaction with bromotrifluoroacetone. "α-bromo-β-(5-imidozoyl) propionic acid, chloroacetyl phosphate, N-alkylmaleimides. 3-nitro-2-pyridyl disulfide, methyl 2-pyridyl disulfide, p-chloromercuribenzoate. 2-chloromercuri-4- nitrophenol, or chloro-7-nitrobenzo-2-oxa-1.3-diazole. '

Histidyl residues are derivatized by reaction with diethylpyrocarbonate at pH 5.5-7.0 because this agent is relatively specific for the histidyl side chain. Para-bromophenacyl bromide also is useful: the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues other suitable reagents for derivatizing an amino-containing residues include imidoesters such as methyl picolinimidate; pyridoxal phosphate: pyridoxal; chloroborohydride; trinitrobenzenesulfonic acid: O-methylisourea: 2,4-pentanedione: and transaminase-catalyzed reaction with glyoxylate.

Arginyl residues are modified by reaction with one or several conventional reagents, among them phenylglyoxal, 2,3- butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I. or ¹³¹I to prepare labeled proteins for use in radioimmunoassay, the chloramine T method described above being suitable.

Carboxyl side groups (aspartyl or glutamyl) are selectively modified by reaction with carbodiimides (R))-N=C=N-R))), where R and R)) are different alkyl groups, such as 1-cyclohexyl-3-(2-morpholinyl-4-ethyl) carbodiimide or 1-ethyl-3-(4-azonia-4.4-dimethylpentyl) carbodiimide. Furthermore, aspartyl and glutamyl residues are converted to asparaginyl and glutaminyl residues by reaction with ammonium ions.

Derivatization with bifunctional agents is useful for cross-linking ErbB ligand to a water-insoluble support matrix or surface. Commonly used cross-linking agents include. e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3.31- maleimides dithiobis(succinimidylpropionate), and bifunctional such as bis-N-maleimido- 1. 8 -octane. Derivatizing agents such as methyl-3-((p-azidophenyl)dithio)propioimidate yield photoactivatable intermediates that are capable of forming cross-links in the presence of light. Alternatively, reactive water-insoluble matrices such as cyanogen bromide-activated carbohydrates and the reactive substrates described in U.S. 3,969,287; 3,691,016;4,195,128; 4,247,642; 4,229.53.7: and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Othermodifications include hydroxylation of proline and lysine, phosphorylation ofhydroxyl groups of seryl or threonyl residues, methylation of the a-amino groups of lysine, arginine, and histidine side chains (T.E. Creighton, Proteins: Structure and Molecular Properties, W.H. Freeman & Co., San Francisco, pp. 79-86 (1983)), acetylation of the N-terminal amine, and amidation of any C-terminal carboxyl group.

ErbB ligand optionally is fused with a polypeptide heterologous to ErbB ligand. The heterologous polypeptide optionally is an anchor sequence such as that found in a phage coat protein such as M 13 gene III or gene VIII proteins. These heterologous polypeptides can be covalently coupled to ErbB ligand polypeptide through side chains or through the terminal residues.

ErbB ligand may also be covalently modified by altering its native glycosylation pattern. One or more carbohydrate substitutents in these embodiments, are modified by adding, removing or varying the monosaccharide components at a given site, or by modifying residues in ErbB ligand as that glycosylation sites are added or deleted.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Glycosylation sites are added to ErbB ligand by altering its amino acid sequence to contain one or more of the above-described tri- peptide sequences (for N-linked glycosylation sites). The alteration may also be made by the addition of, or substitution by, one or more serine or threonine residues to ErbB ligand (for O-linked glycosylation sites). For ease. ErbB ligand is preferably altered through changes at the DNA level, particularly by mutating the DNA encoding ErbB ligand at preselected bases such that codons are generated that will translate into the desired amino acids.

Chemical or enzymatic coupling of glycosides to ErbB ligand increases the number of carbohydrate substituents. These procedures are advantageous in that they do not require production of the polypeptide in a host cell that is capable of N- and O-linked glycosylation. Depending on the coupling mode used, the sugar(s) may be attached to (a) arginine and histidine, (b) free carboxyl groups, (c) free sulfydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, published 11 September 1987, and in Aplin and Wriston (CRC Crit. Rev. Biochem., pp. 259-306 (1981)).

Carbohydrate moieties present on an ErbB ligand also are removed chemically or enzymatically. Chemical deglycosylation requires exposure of the polypeptide to the compound trifluoromethanesulfonic acid, or an equivalent compound. This treatment results in the cleavage of most or all sugars except the linking sugar (N-acetylglucosamine or N-acerylgalactosamine), while leaving the polypeptide intact. Chemical deglycosylation is described by Hakimuddin et al. (Arch. Biochem. Biophys., 259:52 (1987)) and by Edge et al. (Anal. Biochem., 118:131 (1981)). Carbohydrate moieties are removed from ErbB ligand by a variety of endo- and exo- glycosidases as described by Thotakura et al. (Meth. Enzymol., 138:350 (1987)).

Glycosylation also is suppressed by tunicamycin as described by Duksin et al. (J. Biol. Chem., 257:3105 (1982)). Tunicamycin blocks the formation of protein-N-glycoside linkages.

ErbB ligand may also be modified by linking ErbB ligand to various nonproteinaceous polymers, e.g., polyethylene glycol, polypropylene glycol or polyoxyalkylenes, in the manner set forth in U.S. 4,640,835; 4.496,689; 4,301,144; 4,670.417; 4,791,192 or 4,179.337. One preferred way to increase the *in vivo* circulating half life of non-membrane bound ErbB ligand is to conjugate it to a polymer that confers extended half-life, such as polyethylene glycol (PEG). (Maxfield, et al, Polymer 16,505-509 (1975); Bailey, F. E.. et al, in Nonionic Surfactants (Schick. M. J., ed.) pp.794821, 1967); (Abuchowski. A. et al., J. Biol. Chem. 252. 3582-3586, 1977; Abuchowski, A. et al., Cancer Biochem. Biophys. 7, 175-186. 1984); (Katre, N.V. et al., Proc. Natl. Acad. Sci., 84, 1487-1491, 1987; Goodson, R. et al., Bio Technology, 8,343-346, 1990). Conjugation to PEG also has been reported to have reduced immunogenicity and toxicity (Abuchowski, A. et al., J. Biol. Chem., 252, 3578-3581, 1977).

ErbB ligand may also be entrapped in microcapsules prepared, for example, by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

### B. Anti-ErbB Receptor Antibody Preparation

The antibodies contemplated for use in this invention include polyclonal antibodies, monoclonal antibodies and fragments thereof. Preferably, the antibodies employed in the methods of the invention comprise ErbB receptor agonist antibodies which induce or stimulate proliferation of beta precursor cells or mature beta cells, or induce or stimulate differentiation of beta precursor cells.

Polyclonal antibodies are preferably raised in animals by multiple subcutaneous (sc) or intraperitoneal (ip) injections of the relevant antigen (i.e., an ErbB receptor) and an adjuvant. It may be useful to conjugate the relevant antigen to a protein that is immunogenic in the species to be immunized. *e.g.,* keyhole limpet hemocyanin, serum albumin. bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, and succinic anhydride.

Animals may be immunized against the antigen, immunogenic conjugates, or derivatives by combining, *e.g.,* 100 g or 5 g of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies may be made using the hybridoma method first described by Kohler et al., Nature, 256:495 (1975), or may be made by recombinant DNA methods (U.S. Patent No. 4,816.567).

In the hybridoma method, a mouse or other appropriate host animal, such as a hamster or macaque monkey, is immunized as herein above described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOP-21 and M.C.-11 mouse tumors available from the Salk Institute Cell Distribution Center, San Diego, California USA, and SP-2 or X63-AgB-653 cells available from the American Type Culture Collection. Manassas, Virginia USA. Human myeloma and mouse-human heteromyeloma cell lines also have been described for the production of human monoclonal antibodies (Kozbor. J. Immunol., 133:3001 (1984): Brodeur et al., Monoclonal Antibody Production Techniques and Applications, pp. 51-63 (Marcel Dekker, Inc., New York. 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in *vitro* binding assay, such as radioimmunoassay (RIA) or enzyme-linked immunoabsorbent assay (ELISA).

The binding affinity of the monoclonal antibody can, for example, be determined by the Scatchard analysis of Munson et al.. Anal, Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles und Practice. pp.59-103 (Academic Press. 1986)). Suitable culture media for this purpose include, for example. D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-SEPHAROSE. hydroxylapatite chromatography, gel electrophoresis, dialysis, or affinity chromatography.

DNA encoding the monoclonal antibodies is readily isolated and sequenced using conventional procedures (e.g.. by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells. Chinese hamster ovary (CHO) cells, or myeloma cells that do not otherwise produce immunoglobulin protein, to obtain the synthesis of monoclonal antibodies in the recombinant host cells.

Hybridoma cell lines producing antibodies may be identified by screening the culture supernatants for antibody which binds to one or more ErbB receptors. This is routinely accomplished by conventional immunoassays using soluble receptor preparations or by FACS using cell-bound receptor and labeled candidate antibody. ErbB receptor agonist antibodies are preferably antibodies which activate ErbB receptor phosphorylation, which can be determined using known tyrosine phosphorylation assays in the art. Certain agonist antibodies to one or more ErbB receptors have been previously described, for instance, by Yarden, Proc. Natl. Acad. Sci. 87:2569-2573 (1990) and Defize et al., EMBO J. 5:1187-1192 (1986).

The hybrid cell lines can be maintained in culture *in vitro* in cell culture media. The cell lines of this invention can be selected and/or maintained in a composition comprising the continuous cell line in hypoxanthine-aminopterin thymidine (HAT) medium. In fact, once the hybridoma cell line is established, it can be maintained on a variety of nutritionally adequate media. Moreover, the hybrid cell lines can be stored and preserved in any number of conventional ways, including freezing and storage under liquid nitrogen. Frozen cell lines can be revived and cultured indefinitely with resumed synthesis and secretion of monoclonal antibody. The secreted antibody is recovered from tissue culture supernatant by conventional methods such as precipitation, ion exchange chromatography, affinity chromatography, or the like. The antibodies described herein are also recovered from hybridoma cell cultures by conventional methods for purification of IgG or IgM as the case may be that heretofore have been used to purify these immunoglobulins from pooled plasma, e.g.. ethanol or polyethylene glycol precipitation procedures.

Human antibodies may be used. Such antibodies can be obtained by using human hybridomas (Cole et al., Monoclonal Antibodies and Cancer Therapy, Alan R. Liss. p. 77 (1985)). Chimeric antibodies. Cabilly et al., U.S. 4.816.567. (Morrison et al., Proc. Natl. Acad. Sci., 81:6851 (1984): Neuberger et al., Nature 312:604 (1984); Takeda et al., Nature 314:452 (1985)) containing a murine variable region and a human constant region of appropriate biological activity (such as ability to activate human complement and mediate ADCC) are within the scope of this invention, as are humanized antibodies produced by conventional CDR-grafting methods (Riechmann et al., Nature 332:333-327(1988); EP 0328404 A1: EP 02394000 A2).

Techniques for creating recombinant DNA versions of the antigen-binding regions of antibody molecules (Fab or variable regions fragments) which bypass the generation of monoclonal antibodies are also encompassed within the practice of this invention. One extracts antibody-specific messenger RNA molecules from immune system cells taken from an immunized subject, transcribes these into complementary DNA (cDNA), and clones the cDNA into a bacterial expression system and selects for the desired binding characteristic. The Scripps/Stratagene method uses a bacteriophage lambda vector system containing a leader sequence that causes the expressed Fab protein to migrate to the periplasmic space (between the bacterial cell membrane and the cell wall) or to be secreted.

One can rapidly generate and screen great numbers of functional Fab fragments to identify those which bind the receptors with the desired characteristics. Alternatively, the antibodies can be prepared by the phage display techniques described in Hoogenboom. Tibtech February 1997 (vol 15): Neri et al., Cell Biophysics 27:47-61 (1995); Winter et al., Annu. Rev. Immunol., 12:433-55 (1994); and Soderlind et al., Immunol. Rev. 130:109-124 (1992) and the references described therein as well as the monovalent phage display technique described in Lowman et al., Biochem., 30:10832-10838 (1991).

### C. Therapeutic Compositions and Methods

The ErbB receptor ligands or ErbB receptor agonist antibodies may be employed to induce or stimulate mature beta cell or precursor beta cell proliferation. The ErbB receptor ligands or ErbB receptor agonist antibodies may also be employed to induce or stimulate beta precursor cell differentiation. Use of ErbB ligands is, in particular, referred to in the methods below.

The uses of the invention include uses for treating pancreatic dysfunction in mammals. A preferred method is a method of treating diabetes, and even more preferably, Type I diabetes. It is contemplated that an ErbB receptor ligand may be administered as a single therapeutic agent in treating the mammal in need of such treatment. Alternatively, an ErbB ligand may be administered in combination with one or more other ErbB ligands. For instance, the mammal may be administered a combination of both heregulin and betacellulin. It is further contemplated that the ErbB receptor ligands may be administered in combination with other therapies or agents useful for treating pancreatic dysfunction, or symptoms associated with such pancreatic dysfunction, such as insulin, sulphonylurea, cyclosporin or other known immunosuppressive agents, thiozolidenediones, and metformin.

Compositions, such as pharmaceutically acceptable formulations, can be prepared for storage by mixing the ErbB receptor ligand having the desired degree of purity with optional carriers, excipients, or stabilizers (Remington's Pharmaceutical Sciences, *supra).* Acceptable carriers, excipients or stabilizers should be nontoxic to recipients at the dosages and concentrations employed, and include buffers such as phosphate, citrate, and other organic acids; antioxidants including ascorbic acid; low molecular weight (less than about 10 residues) polypeptides: proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA: sugar alcohols such as mannitol or sorbitol; salt-forming counterions such as sodium: and/ornonionic surfactants such as TWEEN, PLURONICS or polyethylene glycol (PEG).

The ErbB receptor ligand to be used for *in vivo* administration should be sterile. This is readily accomplished by filtration through sterile filtration membranes, prior to or following lyophilization and reconstitution. The ligand optionally will be stored in lyophilized form or in solution.

Therapeutic compositions containing the ErbB receptor ligand(s) generally are placed into a container having a sterile access port, for example, an intravenous solution bag or vial having a stopper pierceable by a hypodermic injection needle.

The route of administration will usually be in accord with known methods, e.g., injection or infusion by intravenous, intraperitoneal, intracerebral, intramuscular, intraocular, intraarterial, powder or liquid aerosol administration, or by sustained release systems as noted below. The selected ErbB receptor ligand may be administered continuously by infusion or by bolus injection. It is contemplated that the ErbB receptor ligand may be administered to the mammal via a cannula, such as by inserting a cannula device into the pancreas or pancreatic tissue. The cannula device may also be employed to administer the ErbB receptor ligand to the mammal via the celiac artery. The use of a cannula device for delivery of therapeutic agent is known in the art and may be accomplished using techniques known to the skilled artisan.

Suitable examples of sustained-release preparations include semipermeable matrices of solid hydrophobic polymers containing the ErbB receptor ligand, which matrices are in the form of shaped articles, e.g. films, or microcapsules. Examples of sustained-release matrices include polyesters, hydrogels (e.g., poly(2-hydroxyethylmethacrylate) as described by Langer et al., J. Biomed. Mater. Res., 15: 167-277 (1981) and Langer, Chem. Tech., 12:98-105 (1982) or poly(vinylalcohol)), polylactides (U.S. 3,773,919. EP 58.481), copolymers of L-glutamic acid and gamma ethyl-L-glutamate (Sidman et al., Biopolymers, 22: 547-556 (1983)), non-degradable ethylene-vinyl acetate (Langer *et al., supra),* degradable lactic acid-glycolic acid copolymers such as the LUPRON DEPOT (injectable microspheres composed of lactic acid-glycolic acid copolymer and leuprolide acetate), and poly-D-(-)-3-hydroxybutyric acid (EP 133.988). While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulthydryl residues, lyophilizing from acidic solutions, controlling moisture content, using appropriate additives, and developing specific polymer matrix compositions.

Sustained-release compositions also include liposomally entrapped ErbB receptor ligand. Liposomes containing the selected ligand may be prepared by methods known *per se:* DE 3,218,121; Epstein et al.. Proc. Natl. Acad. Sci. USA, 82:3688-3692 (1985); Hwang et al., Proc. Natl. Acad. Sci. USA, 77:4030-4034 (1980); EP 52,322; EP 36.676: EP 88.046: EP 143.949: EP 142.641; Japanese patent application 83-118008: U.S. 4.485,045 and 4.544.545: and EP 102.324. Ordinarily the liposomes are of the small (about 200-800 Angstroms) unilamelar type in which the lipid content is greater than about 30 mol. % cholesterol, the selected proportion being adjusted for the optimal therapy. Liposomes with enhanced circulation time are disclosed in U.S. patent no. 5.013.556.

An effective amount of ErbB ligand to be employed therapeutically will depend, for example, upon the therapeutic objectives, the route of administration, and the condition of the patient, for instance, the severity of the pancreatic dysfunction. It may be necessary for the therapist to titer the dosage and modify the route of administration as required to obtain the optimal therapeutic effect. A typical daily dosage might range from about 1µg/kg to about 1 mg/kg and up to 100 mg/kg or more, depending on the factors mentioned above. Typically, the clinician will administer the selected polypeptide(s) until a dosage is reached that achieves the desired effect. Making the determinations of dosing and scheduling is within the routine skill of the art. Effective doses may be extrapolated from dose-response curves derived from *in vitro* or animal model test systems. The progress of this therapy is easily monitored by conventional assays, for example, by testing for c-peptide levels, glucose-tolerance testing, or blood analysis of glucose levels.

The invention also provides methods of *ex vivo* treatment of mammalian cells using ErbB ligand. Such *ex vivo* treatment may be useful in treating, for instance, beta precursor cells in culture, and subsequently transplanting the treated cells into a mammal in need of such treatment using appropriate transplantation techniques. Optionally, the transplantation is an autologous transplantation wherein the mammal's own cells are removed, treated in culture with ErbB ligand, and then to be transplanted back to the same mammal.

In the methods cells or tissue(s) containing mature beta cells or beta precursor cells may be obtained from a mammal (such as by performing a surgical or biopsy procedure), and preferably are obtained aseptically. The number of cells or amount of tissue needed for the in *vitro* culture can be determined empirically. The cells or tissues are then placed in a suitable cell or tissue culture dish or plate and exposed to one or more ErbB ligands. Typically, the ErbB ligand will be added to the cell culture at a concentration of about 0.1 to about 100 nM, preferably about 1 to 50 nM. If desired, the cells may be cultured for several generations in order to sufficiently expand the beta cell population. Various cell culture mediums known in the art will be suitable for the in *vitro* culture, including Ham's F10. MEM. RPMI 1640. and DMEM. Such media is available from Sigma (St. Louis. MO) and GIBCO (Grand Island, NY). Typically, the culture medium will contain components such as carbohydrates (like glucose), essential amino acids, vitamins, fatty acids, trace elements, and optionally, serum from a mammalian source. The cell culture conditions should be suitable to effect proliferation and/or differentiation of the beta cells.

The treated cells or tissue(s) can be formulated, if desired, in a carrier such as those described above. The treated cells or tissue(s) can then be infused or transplanted into a recipient mammal using techniques known in the art. The recipient mammal may be the same individual as the donor mammal, or may be another heterologous mammal. An "effective amount" of the treated cells or tissue(s) to be transplanted to the mammal will depend, for example, on the therapeutic objectives, the route of administration, and the condition of the patient. It will be within the ordinary skill of the practitioner to determine dose of administration and modify means of administration to obtain the optimal therapeutic effect. Single or multiple doses of the treated cells or tissue(s) may be administered to the recipient mammal. It may be desirable to determine approximate dose ranges *in vitro* or in animal models, from which dose ranges for human patients can be extrapolated. In the methods where heterologous cells or tissue(s) are to be transplanted into the recipient mammal, immunosuppressant agents known in the art, such as cyclosporin, will also typically be administered to the recipient mammal.

Subsequent to the transplantation of the treated cells into the mammal, it is contemplated that further or continued administration of one or more ErbB ligands to the mammal *in vivo* may be useful to further enhance, for example, insulin secretion by the beta cells. Such further or continued administration of ErbB ligand may be accomplished using the compositions and methods described above.

### EXAMPLES

### EXAMPLE 1

Primary cultures of murine fetal pancreatic cells were assayed with various ErbB ligands and the expression of various markers or insulin was examined.

Pancreata were dissected from e 14 embryos of CD1 mice (Charles River Laboratories). The pancreata were then digested with 1.37 mg/ml collogenase/dispase (Boehringer Mannheim) in F12/DMEM (Gibco) at 37°C for 40 to 60 minutes. Following the incubation, the digestion was neutralized with an equal volume of 5% BSA, and then the cells were washed once with RPMI1640 (Gibco).

On Day 1, the cells were seeded into 12-well tissue culture plates that had been precoated with 20 microgram/ml laminin (Boehringer Mannheim) in PBS. Cells from the pancreata of 1-2 embryos were distributed per each well in primary culture medium (RPMI1640 containing 10 microgram/ml rhInsulin (Genentech, Inc.), 50 microgram/ml aprotinin (Boehringer Mannheim), 60 microgram/ml bovine pituitary extract (BPE) (Pel-Freeze), 100 ng/ml Gentamycin, at 1:1000. in 10 ml PBS, 10 microgram/ml Transferrin (Sigma), 10 ng/ml EGF (BRL), 10 microliter of 5X10⁻⁹ M triiodothyronine (Sigma). 100 microliter of 10 nM ethanolamine (Sigma), and at 1:1000, and in 10 ml 200 proof ETOH. 2 microliter of 1 nM hydrocortisone (Sigma), 100 microliter of 10 nM progesterone (Sigma) and 500 microliter of 1 micromolar forskolin (Calbiochem). The cell cultures were then incubated at 37° C.

On Day 2, the primary culture media was removed and the attached cells were washed with RPMI1640. Two ml of minimal media (RPMI1640 containing 10 microgram/ml transferrin, 1 microgram/ml insulin, 100 ng/ml Gentamycin. 50 microgram/ml aprotinin, 1 microgram/ml BPE) was then added, along with the following ErbB receptor ligands (recombinant human forms): EGF, HG-EGF, TGF-alpha, amphiregulin, betacellulin, and heregulin. The heregulin polypeptide (Genentech. Inc.) consisted of the EGF domain only (HRG-beta 1₁₇₇₋₂₄₄). The other ligands consisted of the full length human polypeptide and were purchased from R & D Systems. The respective ligands were added to the cultures at four different concentrations - 200 ng/ml. 100 ng/ml. 20 ng/ml and 4 ng/ml.

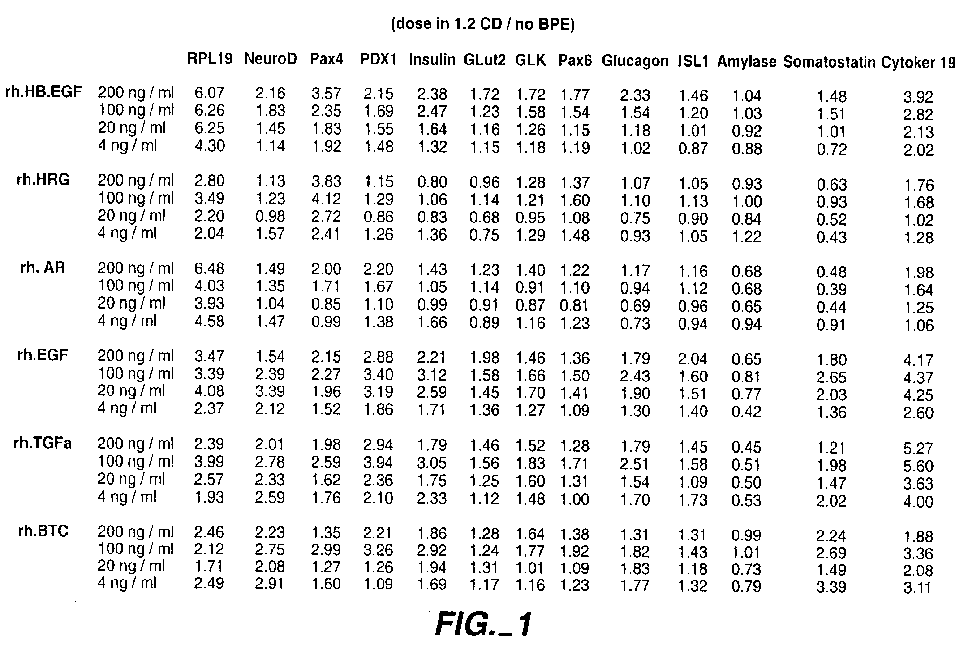

On Day 4, the media was removed from the wells, mRNA was prepared from the cells and assayed for the expression level of the markers identified in Figure 1. The markers are described further in Edlund, Diabetes 47:1817-1823 (1998) and Bouwens, J. Pathology 184:234-239 (1998), and the references cited therein. The mRNA readouts were used to indicate changes in the number of cells expressing the various markers relative to precursor or mature phenotype. Marker expression was analyzed by real-time quantitative RT-PCR. [Gibson et al., Genome Research 6:986-994 (1996)]. An ErbB ligand was determined to be positive if it resulted in an increase in expression of the relevant marker.

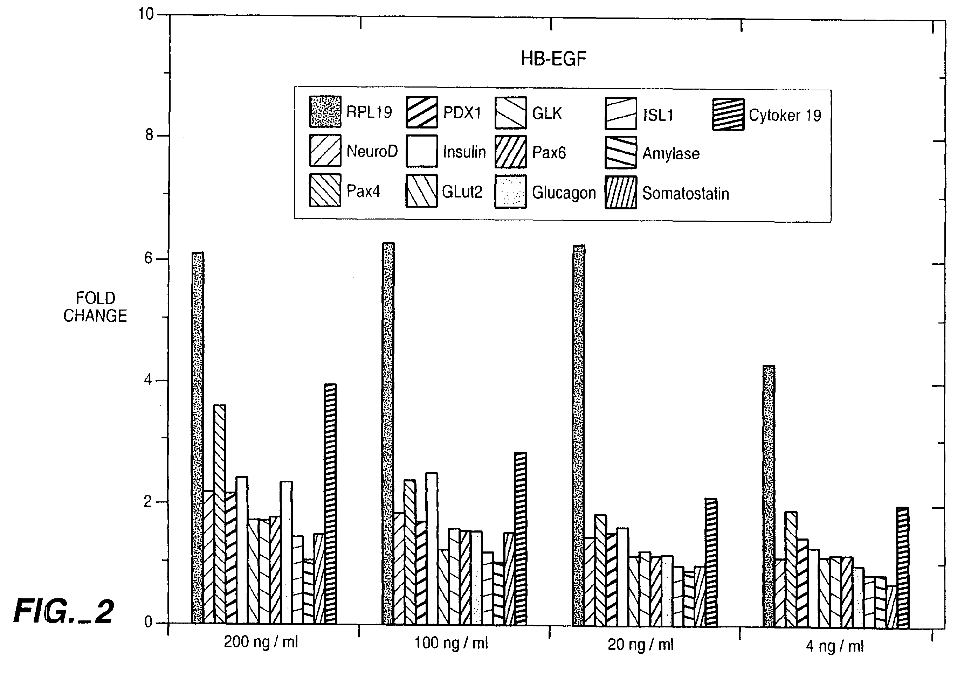

The results are shown in Figure 1. As shown, expression of the markers - RPL19, NeuroD, Pax4, PDX-1, Insulin, Glut2, GLK, Pax6, Glucagon, ISL1, Amylase, Somatostatin, Cytoker 19 - was determined. The results observed for each of the respective ligands are also illustrated graphically in bar diagrams in Figures 2 (HB-EGF), 3 (heregulin), 4 (amphiregulin), 5 (EGF), 6 (TGF-alpha), and 7 (betacellulin).

All of the ErbB ligands tested altered the expression of one or more of the markers. All of the ligands except heregulin produced more than a doubling of PDX-1 expression over a 48 hour exposure to ligand. All of the ligands except betacellulin produced a more than two fold increase in Pax4 expression, and in the presence of all but heregulin, there was a more than two fold increase in insulin expression. None of the ligands tested increased amylase expression, and amphiregulin, EGF, and TGF-alpha actually decreased the level of expression of the amylase marker.

### EXAMPLE 2

Mice heterozygous (+/-) for either heregulin, ErbB2 or ErbB3 were created by gene targeting techniques, resulting in the loss of one functional gene copy and an associated decrease in targeted protein. The *in vivo* activity of heregulin in the heterozygous mouse lines and in wild type mice (pregnant and non-pregnant) was then examined.

The chimeric mice were generated by gene targeting, described in Erickson et al., Development 124:4999-5011 (1997). The mice were mated on C57BL/6J and Balb/C mouse strains with no differences noted in heregulin response based on background strain or backcross level. Adult 8-12 week old mice of each genotype, with an average weight of 20 g each, were treated with a sustained 14 day systemic delivery of recombinant human heregulin-beta 1 (amino acids 177-244) using ALZA pumps. [Holmes et al., Science 256:1205-1210 (1992)]. Genotypic groups receiving the heregulin consisted of 6 females and 6 males each. Control groups for each genotype (2 females and 3 males) received PBS (Gibco). ALZA mini-osmotic pumps (model 2002: pumping rate:0.5 microliter/hour; duration: 14 days; reservoir volume: 200 microliter) were filled according to manufacturer instructions, with the heregulin diluted in PBS and doses were delivered to the animals at 0.75 mg/kg/day or 1.0 mg/kg/day. The pumps were stored at 4°C overnight in PBS prior to sterile implantation. The animals were anesthetized with Ketamine, 75-80 mg/kg, Xylazine, 7.5-15 mg/kg, and Acepromazine, 0.75 mg/kg, delivered intraperitoneally. The filled pump, delivery portal first, was inserted into a subcutaneous pocket along the back. Animals were individually housed and observed daily. Any moribund animals were immediately sacrificed and necropsied.

Surviving animals were sacrificed and necropsied at day 14. Organ tissue was fixed in 10% neutral buffered formalin at room temperature overnight followed by storage in 70% ethanol. For paraffin embedding, tissues were dehydrated through graded alcohols, followed by methyl salicylate and overnight infiltration in Paraplast at 57° C. Serial 6 micrometer sections were cut and affixed to poly-lysine coated slides prior to hematoxylin/eosin staining and histological analysis.

Tolerance for the heregulin treatment varied depending upon genotype. Mortality differed between the genotypes (p<0.001) with the heregulin (+/-) animal groups having the highest mortality (12/12= 100%), the ErbB2 and ErbB3 (+/-) animal groups having the lowest mortality (1/12=8%) and the wild type group having intermediate mortality (7/12=58%). There was no apparent difference in mortality by sex. Both wild type and heregulin (+/-) animals receiving heregulin treatment exhibited lacrimation, dehydration, hunching ruffled fur, a cool body temperature and noticeably hypoactive. The wild type and heregulin (+/-) animals also appeared to have enlarged abdominal regions. The control animals receiving PBS survived the full 14 days with no clinical signs.

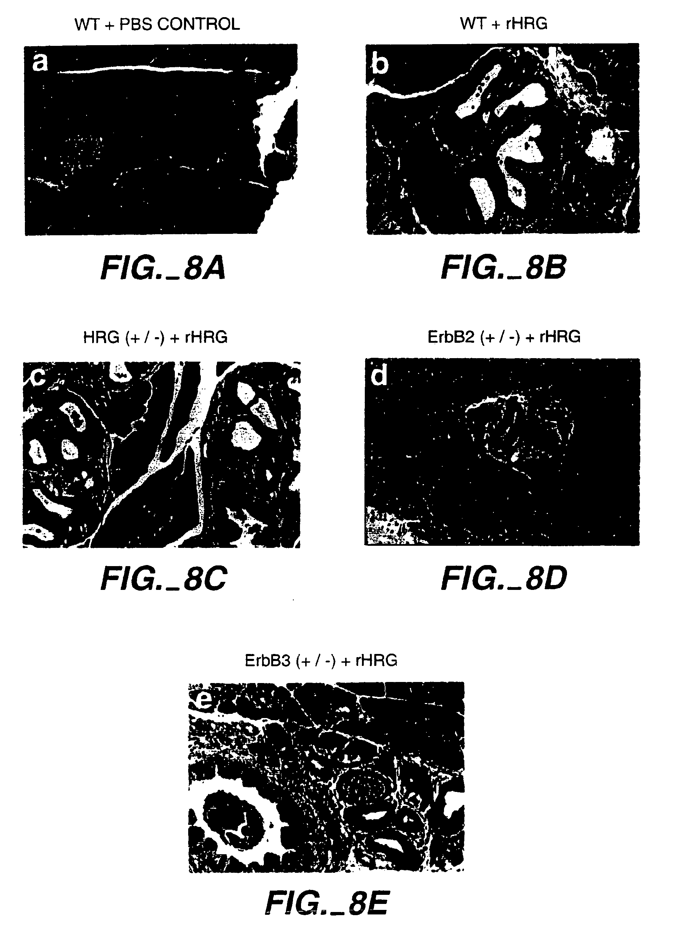

The pancreas appeared largely normal in the treated non-surviving wild type and heregulin (+/-) mice at necropsy, with limited ductal ectasia and minimal hyperplasia probably reflecting the short exposure time of the animals to the administered heregulin (5-6 days) (see Figures 8(b) and 8(c)). In contrast, in the ErbB2 and ErbB3 (+/-) animals receiving treatment for the full 14 days, there was pronounced ductal hyperplasia and proliferation in the main pancreatic ducts at necropsy with inflammatory cells present in the lumen of the ducts (see Figures 8(d) and 8(e)). Acinar cell injury was not widespread, although amylase levels were elevated in many of the animals.

## Claims

1. Use of heregulin in the preparation of a medicament for treating pancreatic dysfunction in a mammal by inducing or stimulating the differentiation or proliferation of beta precursor cells or proliferation of mature beta cells.

2. The use of claim 1, wherein medicament is for treating diabetes in a mammal.

3. The use of claim 1 or claim 2, wherein said heregulin is for administration in combination with betacellulin.

4. The use of claim 2, wherein said diabetes is Type I diabetes.

5. The use of claim 2, wherein said medicament is formulated for administration to said mammal using a cannula.

6. A method of preparing a medicament for treating pancreatic dysfunction, comprising the steps of exposing, *in vitro,* mature beta cells or beta precursor cells from a donor mammal to an effective amount of heregulin in order to induce or stimulate the differentiation or proliferation of said beta precursor cells or the proliferation of said mature beta cells and subsequently formulating said mature beta cells or beta precursor cells for administration to a recipient mammal *in vivo.*

7. The method of claim 6, wherein said donor mammal and said recipient mammal are the same mammal.

8. The method of claim 6, wherein said donor mammal and said recipient mammal are different mammals.

9. The method of claim 8, wherein said mature beta cells or beta precursor cells are formulated for administration in combination with an immunosuppressant agent.

10. A method of stimulating or inducing proliferation of beta precursor cells or mature beta cells, *in vitro,* comprising exposing said beta precursor cells or mature beta cells to an effective amount of heregulin.

11. A method of stimulating or inducing differentiation of beta precursor cells into mature beta cells, *in vitro,* comprising exposing said beta precursor cells to an effective amount of heregulin.

12. A composition comprising an effective amount of heregulin and betacellulin, and a pharmaceutically acceptable carrier.

## Patentansprüche

1. Verwendung von Heregulin zur Herstellung eines Medikaments zur Behandlung von Pankreas-Dysfunktion bei einem Säugetier durch Induktion oder Stimulation der Differenzierung oder Proliferation von β-Vorläuferzellen oder Proliferation reifer β-Zellen.

2. Verwendung nach Anspruch 1, worin das Medikament zur Behandlung von Diabetes bei einem Säugetier dient.

3. Verwendung nach Anspruch 1 oder Anspruch 2, worin das Heregulin zur Verabreichung in Kombination mit β-Cellulin vorgesehen ist.

4. Verwendung nach Anspruch 2, worin der Diabetes Typ-I-Diabetes ist.

5. Verwendung nach Anspruch 2, worin das Medikament zur Verabreichung an das Säugetier unter Verwendung einer Kanüle formuliert ist.

6. Verfahren zur Herstellung eines Medikaments zur Behandlung von Pankreas-Dysfunktion, umfassend die Schritte des In-vitro-Aussetzens reifer β-Zellen oder β-Vorläuferzellen von einem Spender-Säugetier gegenüber einer wirksamen Menge an Heregulin, um die Differenzierung oder Proliferation der β-Vorläuferzellen oder die Proliferation der reifen β-Zellen zu induzieren oder zu stimulieren, sowie des anschließenden Formulierens der reifen β-Zellen oder β-Vorläuferzellen zur In-vivo-Verabreichung an ein Empfänger-Säugetier.

7. Verfahren nach Anspruch 6, worin das Spender-Säugetier und das Empfänger-Säugetier dasselbe Säugetier sind.

8. Verfahren nach Anspruch 6, worin das Spender-Säugetier und das Empfänger-Säugetier unterschiedliche Säugetiere sind.

9. Verfahren nach Anspruch 8, worin die reifen β-Zellen oder β-Vorläuferzellen zur Verabreichung in Kombination mit einem immununterdrückenden Mittel formuliert sind.

10. Verfahren zur Stimulation oder Induktion der In-vitro-Proliferation von β-Vorläuferzellen oder reifen β-Zellen, umfassend das Aussetzen der β-Vorläuferzellen oder reifen β-Zellen gegenüber einer wirksamen Menge an Heregulin.

11. Verfahren zur Stimulation oder Induktion der In-vitro-Differenzierung von β-Vorläuferzellen in reife β-Zellen, umfassend das Aussetzen der β-Vorläuferzellen gegenüber einer wirksamen Menge an Heregulin.

12. Zusammensetzung, umfassend eine wirksame Menge an Heregulin und β-Cellulin sowie einen pharmazeutisch annehmbaren Träger.

## Revendications

1. Utilisation d'héréguline dans la préparation d'un médicament destiné au traitement d'un dysfonctionnement pancréatique chez un mammifère en induisant ou stimulant la différenciation ou la prolifération de cellules précurseurs de cellules bêta ou bien la prolifération de cellules bêta matures.

2. Utilisation suivant la revendication 1, dans laquelle le médicament est destiné au traitement du diabète chez un mammifère.

3. Utilisation suivant la revendication 1 ou la revendication 2, dans laquelle ladite héréguline est destinée à l'administration en association avec la bêtacelluline.

4. Utilisation suivant la revendication 2, dans laquelle ledit diabète est le diabète de Type I.

5. Utilisation suivant la revendication 2, dans laquelle ledit médicament est formulé pour l'administration audit mammifère au moyen d'une canule.

6. Procédé pour la préparation d'un médicament destiné au traitement d'un dysfonctionnement pancréatique, comprenant les étapes d'exposition, *in vitro,* de cellules bêta matures ou de cellules précurseurs de cellules bêta provenant d'un mammifère servant de donneur à une quantité efficace d'héréguline afin d'induire ou de stimuler la différenciation ou la prolifération desdites cellules précurseurs de cellules bêta ou la prolifération desdites cellules bêta matures, et ensuite de formulation desdites cellules bêta matures ou cellules précurseurs de cellules bêta pour l'administration à un mammifère receveur *in vivo.*

7. Procédé suivant la revendication 6, dans lequel ledit mammifère servant de donneur et ledit mammifère receveur consistent en le même mammifère.

8. Procédé suivant la revendication 6, dans lequel ledit mammifère servant de donneur et ledit mammifère receveur sont des mammifères différents.

9. Procédé suivant la revendication 8, dans lequel lesdites cellules bêta matures ou cellules précurseurs de cellules bêta sont formulées pour l'administration en association avec un agent immunosuppresseur.

10. Procédé pour stimuler ou induire la prolifération des cellules précurseurs de cellules bêta ou des cellules bêta matures, *in vitro*, comprenant l'exposition desdites cellules précurseurs de cellules bêta ou desdites cellules bêta matures à une quantité efficace d'héréguline.

11. Procédé pour stimuler ou induire la différenciation de cellules précurseurs de cellules bêta en des cellules bêta matures, *in vitro*, comprenant l'exposition desdites cellules précurseurs de cellules bêta à une quantité efficace d'héréguline.

12. Composition comprenant une quantité efficace d'héréguline et de bêtacelluline, et un support pharmaceutiquement acceptable.
